# EUROPEAN PATENT APPLICATION

(11) **EP 2 077 122 A1**
(43) Date of publication of application: **08.07.2009**
(21) Application number: 07830615.6
(22) Date of filing: 26.10.2007
(51) Int. Cl.: A61K 45/00, A61K 31/38, A61K 31/381, A61K 31/4155, A61P 1/00, A61P 1/10, A61P 1/12, A61P 43/00, C07D 495/04

(54) **THERAPEUTIC AGENTS FOR IRRITABLE BOWEL SYNDROME**

(30) Priority: 26.10.2006 JP 2006291374; 06.12.2006 JP 2006329436
(71) Applicant: Kyowa Hakko Kirin Co., Ltd., Tokyo 100-8185 (JP)
(72) Inventor: YAMAGATA, Tsuyoshi, 1188 Shimotogari Nagaizumi-cho, Sunto-gun Shizuoka 411-8731 (JP); SHIBATA, Kenji, 6-6, Asahi-machi 3-chome, Machida-shi, Tokyo 194-8533 (JP); NISHIYA, Yoichi, 6-6, Asahi-machi 3-chome, Machida-shi, Tokyo 194-8533 (JP); SEISHI, Takashi, 1-6-1, Ohtemachi, Chiyoda-ku, TOkyo 100-8185 (JP); SAKUMA, Takashi, 1188 Shimotogari Nagaizumi-cho, Sunto-gun Shizuoka 411-8731 (JP)
(74) Representative: Vossius & Partner
(86) International application number: PCT/JP2007/070881
(87) International publication number: WO 2008/050853

(57) **Abstract**

The present invention provides a therapeutic agent for irritable bowel syndrome which comprises, as an active ingredient, a compound having an adenosine uptake inhibitory activity, a therapeutic agent for irritable bowel syndrome which comprises, as an active ingredient, a tricyclic compound represented by formula (I) 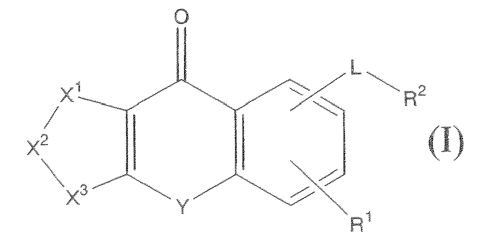 [wherein L represents -NHC(=O)- or the like,
R¹ represents a hydrogen atom, halogen, or the like,
X¹-X²-X³ represents S-CR⁷=CR⁸ (wherein R⁷ and R⁸ may be the same or different and each represents a hydrogen atom, halogen, substituted or unsubstituted lower alkyl, or the like), or the like,
Y represents -CH₂SO₂-, -SO₂CH₂- or the like,
R² represents substituted or unsubstituted lower alkyl, substituted or unsubstituted lower alkoxy, substituted or unsubstituted aryl, or the like] or a pharmaceutically acceptable salt thereof, and the like.

## Description

### Technical Field

The present invention relates to a therapeutic agent for irritable bowel syndrome which comprises, as an active ingredient, a compound having an adenosine uptake inhibitory activity, a therapeutic agent for irritable bowel syndrome which comprises, as an active ingredient, a tricyclic compound or a pharmaceutically salt thereof, or the like.

### Background Art

It is that adenosine is increased in concentration in the blood or tissue under a condition in which cell damage occurs due to decreased oxygen supply such as ischemic, inflammation and so on. It is known that adenosine preceptors are divided into A₁, A_{2A}, A_{2B} and A₃ receptors. Through these receptors, adenosine exhibits various physiological functions such as bradycardia, inhibition of lipolysis, reduction in glomerular filtration, analgesia, reduction in sympathetic and parasympathetic activities, neuronal hyperpolarization, regulation of sensorimotor integration of the basal ganglion, inhibition of platelet aggregation, inhibition of polymorphonuclear leucocytes, vasorelaxation, protection against ischemic damage, stimulation of sensory nerve activity, relaxation of vascular smooth muscle, relaxation of intestinal smooth muscle, inhibition of monocytes and macrophage function, inhibition of cell damage, etc. Since the adenosine receptors are present in the cell membrane, the extracellular adenosine concentration is important for exhibiting physiological functions. Under normal condition, adenosine is intracellularly produced from S-adenosylhomocysteine (SAH) by SAH hydrolase. On the other hand, it is known that under pathologic condition such as ischemia, trauma, stress, or inflammation, adenosine is extracellularly produced from adenosine monophosphate (AMP), which is a metabolite of adenosine triphosphate (ATP), by ecto-5'-nucleotidase. It is known that adenosine is mainly metabolized by intracellular or extracellular adenosine deaminase, but the mechanism of uptake into the cells through the nucleoside transporters plays an important role in a disappearance of extracellular adenosine. The nucleoside transporters are roughly divided into the two types, i.e., equilibrative nucleoside transporters (ENT) and concentrative nucleoside transporters (CNT), and the ENTs are subdivided into the three subtypes of ENT-1, 2, and 3 and the CNTs are subdivided into the three subtypes of the CNT-1, 2, and 3. are expressed in many cells, and adenosine moves inside and outside the cells according to the concentration gradient. Therefore, under a condition, in which adenosine is extracellularly produced, including a pathologic condition, adenosine is taken up into the cells through ENTs. Since adenosine functions through the receptors present extracellularly, the action of the produced adenosine disappears within a short time or the action reduced because the sufficient adenosine concentration cannot be maintained. Although various pharmaceutical effects can be expected by the systemic administration of adenosine or adenosine agonist, undesirable effects are observed at the same time. Since a compound having an action of inhibiting adenosine uptake by ENT blocking functions only in pathologic tissues in which extracellular adenosine production is enhanced, the compound is likely to be used as a useful preventive/therapeutic agent for a certain type of disease condition, [European Journal of Pharmacology, Vol. 495, p.1 (2004)].

On the other hand, irritable bowel syndrome (IBS), one of functional bowel disorders, is a syndrome characterized by abdominal discomfort or pain associated with defecation and Abnormal bowel movement in spite of the absence of a detectable intestinal organic disease. The etiology of IBS is known to be concerned with gastrointestinal motor disorder, altered perception in the bowels, and psychosocial factor (stress). The symptoms include diarrhea, an abdominal pain, an abdominal bloating, and constipation, and are classified into a diarrhea type, a constipation type, and an alternating diarrhea and constipation type according to main symptoms. The symptoms may be accompanied with a psychological condition such as anxiety, hypersensitivity, tension, fretfulness, depression, or the like. The gastrointestinal function is highly regulated by the nerves, and a variety of receptors are present. Therefore, an anticholinergic agent, an antidiarrheal, or a laxative is administered according to the gastrointestinal symptoms such as an abdominal pain, diarrhea, and constipation, and an antidepressant or an antianxiety agent is used if needed. Although alosetron HCl, a serotonin 5-HT₃ receptor antagonist, is known as a therapeutic agent for diarrhea-type IBS, this agent is applied only to woman patients with severe symptoms because serious gastrointestinal disorder, particularly ischemic colitis and serious constipation, are observed. Further, although tegaserod maleate, a serotonin 5-HT₄ receptor agonist, is known as a therapeutic agent for constipation-type IBS, this agent is applied only to women patients. Further, polycarbophil Ca, a highly-water-absorbing polymer, is known as a therapeutic agent for both the diarrhea-type and constipation-type IBS. However, the therapeutic options of IBS are limited. Therefore, a novel therapeutic agent for IBS with no difference in the effects is currently desired [Reviews in Gastroenterological Disorders, Vol.1, No.1, p.2 (2001), and British Journal of Clinical Pharmacology, Viol.56, p.362 (2003)].

There have been known a therapeutic agent for urinary incontinence (refer to Patent Documents 1 and 2), a therapeutic agent for overactive bladder (refer to Patent Document 3), a therapeutic agent for bladder hypersensitivity (refer to Patent Document 4), a therapeutic agent for bladder irritative symptoms associated with benign prostatic hyperplasia (refer to Patent Document 5), a therapeutic agent for overactive bladder associated with cerebrovascular disease (refer to Patent Document 6), a therapeutic agent for pruritus (refer to Patent document 7), an antitussive (refer to Patent Document 8), a therapeutic agent for pain (refer to Patent Document 9), and an antiasthmatic agent (refer to Patent Document 10) comprising, as an active ingredient, a tricyclic compound or a pharmaceutically acceptable salt thereof.
Patent Document 1: a WO97/14672
Patent Document 2: WO98/46587
Patent Document 3: WO02/078710
Patent Document 4: WO02/078711
Patent Document 5: WO02/078712
Patent Document 6: WO2005/000293
Patent Document 7: WO03/041704
Patent Document 8: WO2004/087131
Patent Document 9: WO2005/007154
Patent Document 10: WO2005/011674

### Disclosure of the Invention

### Problems to be solved by Invention

An object of the present invention is to provide a therapeutic agent for irritable bowel syndrome which comprises, as an active ingredient, a compound having an adenosine uptake inhibitory activity, a therapeutic agent for irritable bowel syndrome which comprises, as an active ingredient, a tricyclic compound or a pharmaceutically acceptable salt thereof, and like.

The present invention relates to (1)-(77).
(1) A therapeutic agent for irritable bowel syndrome which comprises, as an active ingredient, a compound having an adenosine uptake inhibitory activity.
(2) Am antidiarrheal which comprises, as an active ingredient, a compound having an adenosine uptake inhibitory activity.
(3) A laxative which comprises, as an active ingredient, a compound having an adenosine uptake inhibitory activity.
(4) A therapeutic agent for irritable bowel syndrome which comprises, as an active ingredient, a tricyclic compound represented by Formula (I) 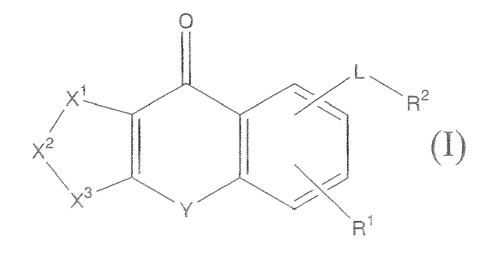 {wherein L represents an oxygen atom, a sulfur atom, -N(R⁹)-(wherein R⁹ represents a hydrogen atom or substituted or unsubstituted lower alkyl), -NHC(=O)- or -C(=O)NH-,
   R¹ represents a hydrogen atom, halogen, substituted or unsubstituted lower alkyl or substituted or unsubstituted lower alkoxy,
   X¹-X²-X³ represents CR⁵=CR⁶-CR⁷=CR⁸ [wherein R⁵, R⁶, R⁷ and R⁸ may be the same or different and each represents a hydrogen atom, halogen, hydroxy, nitro, amino, mono(lower alkyl)-substituted amino, di(lower alkyl)-substituted amino, substituted or unsubstituted lower alkyl, substituted or unsubstituted lower alkoxy, or substituted or unsubstituted (lower alkanoyl)amino],
   N(O)ₘ=CR⁶-CR⁷=CR⁸ (wherein R⁶, R⁷ and R⁸ have the same meanings as defined above, respectively and m represents 0 or 1),
   CR⁵=CR⁶-N(O)ₘ=CR⁸ (wherein R⁵, R⁶, R⁸ and m have the same meanings as defined above, respectively), CR⁵=CR⁶-CR⁷=M(O)ₘ (wherein R⁵, R⁶, R⁷ and m have the same meanings as defined above, respectively), CR⁵=CR⁶-O (whereon R⁵ and R⁶ have the same meanings as defined above, respectively), CR⁵=CR⁶-S (wherein R⁵ and R⁶ have the same meanings as defined above, respectively), O-CR⁷=CR⁸ (wherein R⁷ and
   R⁸ have the same meanings as defined above, respectively),
   S-CR⁷=CR⁸ (whereon R⁷ and R⁸ have the same meanings as defined above, respectively) or O-CR⁷=N (wherein R⁷ has the same meaning as defined above),
   Y represents -CH₂S-, -CH₂SO-, -CH₂SO₂-, -CH₂O-, -CH=CH-, -(CH₂)ₚ-(wherein p represents an integer of 0 to 2), -SCH₂-, -SOCH₂-, -SO₂CH₂- or -OCH₂-, and
   R² represents a hydrogen atom, amino, substituted or unsubstituted lower alkyl, substituted or unsubstituted lower alkenyl, substituted or unsubstituted lower alkoxy, mono(substituted or unsubstituted lower alkyl)-substituted amino, di(substituted or unsubstituted lower alkyl)-substituted amino, substituted or unsubstituted aryl, substituted or unsubstituted heteroaryl, substituted or unsubstituted aralkylamino, substituted or unsubstituted arylamino, or a substituted or unsubstituted heterocyclic group} or a pharmaceutically acceptable salt thereof.
(5) An antidiarrheal which comprises, as an active ingredient, the tricyclic compound or the pharmaceutically acceptable salt thereof described in (4).
(6) A laxative which comprises, as an active ingredient, the tricyclic compound or the pharmaceutically acceptable salt thereof described in (4).
(7) An adenosine uptake inhibitor which comprises, as an active ingredient, the compound or the pharmaceutically acceptable salt thereof described in (4).
(8) A therapeutic agent for irritable bowel syndrome which comprises, as an active ingredient, a tricyclic compound repesented by Formula (Ia) 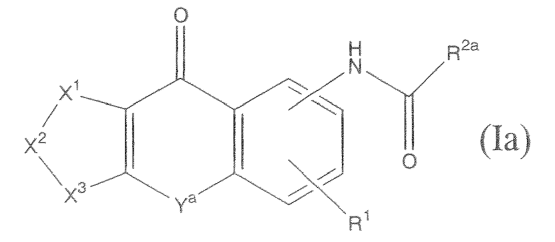 [(wherein R¹ and X¹-X²-X³ have the same meanings as defined above, respectively,
   Y^{a} represents -CH₂SO₂-, -SCH₂-, -SOCH₂-, -SO₂CH₂- or -OCH₂-, and
   when Y^{a} is -CH₂SO₂-, -SCH₂-, -SOCH₂- or -SO₂CH₂-,
   R^{2a} represents a hydrogen atom, amino, substituted or unsubstituted lower alkyl, substituted or unsubstituted lower alkenyl, substituted or unsubstituted lower alkoxy, mono(substituted or unsubstituted lower alkyl)-substituted amino, di(substituted or unsubstituted lower alkyl)-substituted amino, substituted or unsubstituted aryl, substituted or unsubstituted heteroaryl, substituted or unsubstituted aralkylamino, substituted or unsubstituted arylamino, a substituted or unsubstituted heteroalicyclic group, or a substituted or unsubstituted nitrogen-containing heterocyclic group, and
   when Y^{a} is -OCH₂-,
   R^{2a} represents a hydrogen atom, amino, trifluoromethyl, substituted or unsubstituted lower alkenyl, substituted or unsubstituted lower alkoxy, mono(substituted or unsubstituted lower alkyl)-substituted amino, di(substituted or unsubstituted lower alkyl)-substituted amino, substituted or unsubstituted aryl, substituted or unsubstituted heteroaryl, substituted or unsubstituted aralkylamino, substituted or unsubstituted arylamino, a substituted or unsubstituted heteroalicyclic group, a substituted or unsubstituted nitrogen-containing heterocyclic group, or Formula (II) 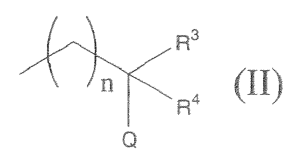 (wherein n is 0 or 1; R³ and R⁴ may be the same or different each represents a hydrogen atom, substituted or unsubstituted lower alkyl, substituted or unsubstituted cycloalkyl, substituted or unsubstituted aryl, or substituted or unsubstituted aralkyl, or R³ and R⁴ may be together with the adjacent carbon atom thereto to form cycloalkyl; and Q represents halogen, amino, hydroxy or substituted or unsubstituted lower alkoxy)] or a pharmaceutically acceptable salt thereof.
(9) The therapeutic agent for irritable bowel syndrome according two (8) wherein Y^{a} is -CH₂SO₂-, -SCH₂-, -SOCH₂- or -SO₂CH₂-.
(10) The therapeutic agent for for irritable bowel syndrome according to (8) wherein Y^{a} is -OCH₂-.
(11) The therapeutic agent for irritable bowel syndrome according to any of (8) to (10) wherein R¹ is a hydrogen atom, halogen or substituted or unsubstituted alkoxy.
(12) The therapeutic agent for irritable bowel syndrome according to any of (8) to (10) wherein R¹ is a hydrogen atom.
(13) The therapeutic agent for irritable bowel syndrome according to of (8), (11) and (12) wherein Y^{a} is -CH₂SO₂-, -SO₂CH₂- or -OCH₂-.
(14) The therapeutic agent for irritable bowel syndrome according to any of (8), (11) any (12) wherein Y^{a} is -CH₂SO₂- or -SO₂CH₂-.
(15) The therapeutic agent for irritable bowel syndrome according to any of (8), (11) and (12) wherein Y^{a} is -CH₂SO₂-.
(16) The therapeutic agent for irritable bowel syndrome according to any of (8) to (15) wherein X¹-X²-X³ is S-CR⁷=CR⁸ (wherein R⁷ and R⁸ have the same as above, respectively).
(17) The therapeutic agent for irritable bowel syndrome according to any of (8) to (15) wherein X¹-X²-X³ is CR⁵=CR⁶-CR⁷=CR⁸ (wherein R⁵, R⁶, R⁷ and R⁸ have the same meanings as defined above, respectively).
(18) The therapeutic agent for irritable bowel syndrome according to any of (8) to (17), wherein R^{2a} is Formula (II) 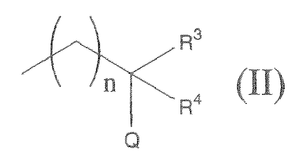 (wherein n, R³, R⁴ and Q have the same meanings as defined above, respectively).
(19) The therapeutic agent for irritable bowel syndrome according to (18), wherein n is 0.
(20) The therapeutic agent for irritable bowel syndrome according to (19), wherein R³ is methyl, R⁴ is trifluoromethyl, and Q is hydroxy.
(21) The therapeutic agent for irritable bowel syndrome according to (8), wherein R¹ is a hydrogen atom, Y^{a} is -CH₂SO₂-, X¹-X²-X³ is S-CR⁷=CR⁸ (wherein R⁷ and R⁸ have the same meanings as defined above, respectively), and R^{2a} is Formula (III) 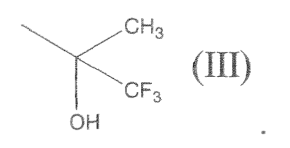
(22) An antidiarrheal which comprises, as an active ingredient, the tricyclic compound or the pharmaceutically acceptable salt thereof described in any of (8) to (21).
(23) A laxative which comprises, as an active ingredient, the tricyclic compound or the pharmaceutically acceptable salt thereof described in any of (8) to (21).
(24) An adenosine uptake inhibitory which comprises, as an active ingredient, the tricyclic compound or the pharmaceutically acceptable salt thereof described in any of (8) to (21).
(25) An therapeutic agent for irritable bowel syndrome which comprises, as an active ingredient, a tricyclic compound represented by Formula (Ib) 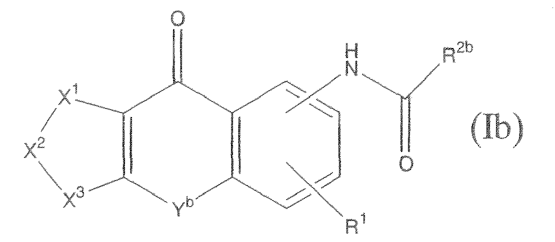 [wherein R¹ and X¹-X²-X³ have the same meanings as defined above, respectively, Y^{b} represents -CH₂O-, -CH₂S-, -CH₂SO-, -CH=CH- or -(CH₂)_{P}- (wherein p has the same meaning as defined above), and R^{2b} represents Formula (III) 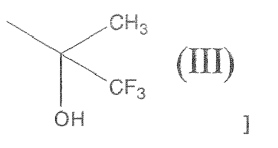 or a pharmaceutically acceptable salt thereof.
(26) The therapeutic agent for irritable bowel syndrome according to (25), wherein X¹-X²-X³ is CR⁵=CR⁶-CR⁷=CR⁸ (wherein R⁵, R⁶, R⁷ and R⁸ have the same meanings as defined above, respectively) or CR⁵=CR⁶-CR⁷=N (wherein R⁵, R⁶ and R⁷ have the same meanings as defined above, respectively).
(27) The therapeutic agent for irritable bowel syndrome according to (25), wherein X¹-X²-X³ is CR⁵=CR⁶-O (wherein R⁵ and R⁶ have the same meanings as defined above, respectively) or CR⁵=CR⁶-S (wherein R⁵ and R⁶ have the same meanings as defined above, respectively).
(28) The therapeutic agent for irritable bowel syndrome according to (25), wherein. X¹-X²-X³ is O-CR⁷=CR⁸ (whereon R⁷ and R⁸ have the same meanings as defined above, respectively) or S-CR⁷=CR⁸ (wherein R⁷ and R⁸ have the same meanings as defined above, respectively).
(29) The therapeutic agent for irritable bowel syndrome according to any of (25) to (28), wherein Y^{b} is -CH₂O-.
(30) The therapeutic agent for irritable bowel syndrome according to any of (25) to (28), wherein Y^{b} is -(CH₂)ₚ- (wherein p has the same meaning as defined above).
(31) The therapeutic agent for irritable bowel syndrome according to (30), wherein p is 0.
(32) The therapeutic agent for irritable bowel syndrome according to (30), wherein p is 2.
(33) The therapeutic agent for irritable bowel syndrome according to any of (25) to (28), wherein Y^{b} is -CH=CH-.
(34) The therapeutic agent for irritable bowel syndrome according to any of (25) to (28), wherein Y^{b} is -CH₂S- or -CH₂SO-.
(35) An antidiarrheal which comprises, as an active ingredient, the tricyclic compound or the pharmaceutically acceptable salt thereof described in any of (25) to (34).
(36) A laxative which comprises, as an active ingredient, the tricyclic compound or the pharmaceutically acceptable salt thereof described in any of (25) to (34).
(37) An adenosine uptake inhibitor which comprises, as an active ingredient, the tricyclic compound or the pharmaceutically acceptable salt thereof described in any of (25) to (34).
(38) A tricyclic compound represented by Formula (Ic) 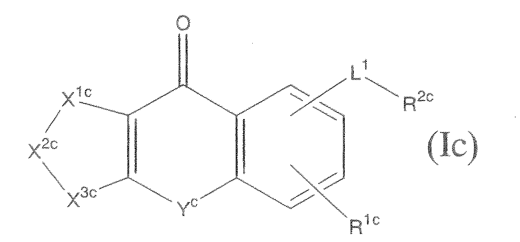 [wherein L¹ represents an oxygen atom or a sulfur atom,
   R^{1c} represents a hydrogen atom or substituted or unsubstituted lower alkyl,
   X^{1c}-X^{2c}-X^{3c} represents O-CR⁷=CR⁸ (wherein R⁷ and R⁸ have the same meanings as defined above, respectively) or S-CR⁷=CR⁸ (wherein R⁷ and R⁸ have the same meanings as defined above, respectively),
   Y^{c} represents -CH₂S-, -CH₂SO- or -CH₂SO₂- and
   R^{2c} represents substituted or unsubstituted lower alkyl] or a pharmaceutically acceptable salt thereof.
(39) The tricyclic compound or the pharmaceutically acceptable salt thereof according to (38), wherein R^{1c} is a hydrogen atom, X^{1c}-X^{2c}-X^{3c} is S-CR^{7c}=CR^{8c} (wherein R^{7c} and R^{8c} may be the same or different and each represents a hydrogen atom or substituted or unsubstituted lower alkyl),
   Y^{c} is -CH₂SO₂- and
   R^{2c} is substituted or unsubstituted benzyl.
(40) A pharmaceutical composition which comprises, as an active ingredient, the tricyclic compound or the pharmaceutically acceptable salt thereof described in (38) or (39).
(41) A therapeutic agent for irritable bowel syndrome which comprises, as an active ingredient, the tricyclic compound or the pharmaceutically acceptable salt thereof described in (38) or (39).
(42) An antidiarrheal which comprises, as an active ingredient, the tricyclic compound or the pharmaceutically acceptable salt thereof, described in (38) or (39).
(43) A laxative which comprises, as an active ingredient, the tricyclic compound or the pharmaceutically acceptable salt thereof, described in (38) or (39).
(44) An adenosine uptake inhibitor which comprises, as an active ingredient, the tricyclic compound or the pharmaceutically acceptable salt thereof described in (38) or (39).
(45) A tricyclic compound represented by Formula (Id) 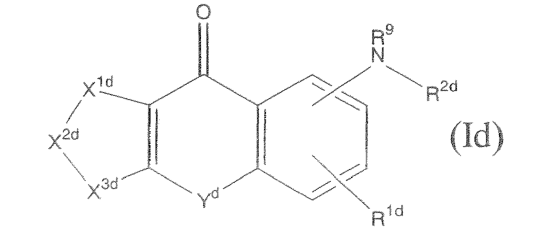 [wherein R⁹ has the same meaning as defined above,
   R^{1d} represents a hydrogen atom or substituted or unsubstituted lower alkyl,
   X^{1d}-X^{2d}-X^{3d} represents O-CR⁷=CR⁸ (wherein R⁷ and R⁸ have the same meanings as defined above, respectively) or S-CR⁷=CR⁸ (wherein R⁷ and R⁸ have the same meanings as defined above, respectively),
   Y^{d} represents -CH₂S-, -CH₂SO-, or -CH₂SO₂-, and
   R^{2d} represents substituted or unsubstituted lower alkyl or substituted or unsubstituted aryl] or a pharmaceutically acceptable salt thereof.
(46) The tricyclic compound or pharmaceutically acceptable salt thereof according to (45) wherein R^{1d} is a hydrogen atom, X^{1d}-X^{2d}-X^{3d} is S-CR^{7d}=CR^{8d} (wherein R^{7d} and R^{8d} may be the same or different and each represents a hydrogen atom or substituted or unsubstituted lower alkyl),
   Y^{d} represents -CH₂SO₂- and
   R⁹ is a hydrogen atom.
(47) The tricyclic compound or the pharmaceutically acceptable salt thereof according to (46), wherein R^{2d} is substituted or unsubstituted lower alkyl.
(48) The tricyclic compound or the pharmaceutically acceptable salt thereof according to (46), wherein R^{2d} is substituted or unsubstituted aryl.
(49) A pharmaceutical composition which comprises, as an active ingredient, the tricyclic compound or the pharmaceutically acceptable salt thereof described in any of (45) to (48).
(50) A therapeutic agent for irritable bowel syndrome which comprises, as an active ingredient, the tricyclic compound or the pharmaceutically acceptable salt thereof described in any of (45) to (48).
(51) An antidiarrheal which comprises, as an active ingredient, the tricyclic compound or the pharmaceutically acceptable salt thereof described in any of (45) to (48).
(52) A laxative which comprises, as an active ingredient, the tricyclic compound or the pharmaceutically acceptable salt thereof described in any of (45) to (48).
(53) An adenosine uptake inhibitor which comprises, as an active ingredient, the tricyclic compound or the pharmaceutically acceptable salt thereof described in any of (45) to (48).
(54) A method for treating irritable bowel syndrome which comprises a step of administering an effective amount of a compound having an adenosine uptake inhibitory activity.
(55) A method for treating diarrhea which comprises a step of administering an effective amount of a compound having an adenosine uptake inhibitory activity.
(56) A method for treating constipation which comprises a step of administering an effective amount of a compound having an adenosine uptake inhibitory activity.
(57) A method for treating irritable bowel syndrome which comprises a step of administering an effective amount of the tricyclic compound described in any of (4), (8) to (21) and (25) to (34).
(58) A method for treating diarrhea which comprises a step of administering an effective amount of the tricyclic compound described in any of (4), (8) to (21) and (25) to (34).
(59) A method for treating constipation which comprises a step of administering an effective amount of the tricyclic compound described in any of (4), (8) to (21) and (25) to (34).
(60) A method for treating irritable bowel syndrome which which comprises a step of administering an effective amount of the tricyclic compound described in (38) or (39).
(61) A method for treating diarrhea which comprises a step of administering an effective amount of the tricyclic compound described in (38) or (39).
(62) A method for treating constipation which comprises a step of administering an effective amount of the tricyclic compound described in (38) or (39).
(63) A method for treating irritable bowel syndrome which comprises a step of administering an effective amount of the tricyclic compound described in any of (45) to (48).
(64) A method for treating diarrhea which comprises a step of administering an effective amount of the tricyclic compound described in any of (45) to (48).
(65) A method for treating constipation which comprises a step of administering an effective amount of the tricyclic compound described in any of (45) to (48).
(66) Use of a compound having an adenosine uptake inhibitory activity for the manufacture of a therapeutic agent for irritable bowel syndrome.
(67) Use of a compound having an adenosine uptake inhibitory activity for the manufacture of an antidiarrheal.
(68) Use of a compound having an adenosine uptake inhibitory activity for the manufacture of a laxative.
(69) Use of the tricyclic compound described in any of (4), (8) to (21) and (25) to (34) for the manufacture of a therapeutic agent for irritable bowel syndrome.
(70) Use of the tricyclic compound described in any of (4), (8) to (21) and (25) to (34) for the manufacture of an antidiarrheal.
(71) Use of the tricyclic compound described in any of (4), (8) to (21) and (25) to (34) for the manufacture of a laxative.
(72) Use of the tricyclic compound described in (38) or (39) for the manufacture of a therapeutic agent for irritable bowel syndrome.
(73) Use of the tricyclic compound described in (38) or (39) for manufacture of an antidiarrheal.
(74) Use of the tricyclic compound described in (38) or (39) for the manufacture of a laxative.
(75) Use of the tricyclic compound described in any of (45) to (48) for the manufacture of a therapeutic agent for irritable bowel syndrome.
(76) Use of the tricyclic compound described in any of (45) to (48) for the manufacture of an antidiarrheal.
(77) Use of the tricyclic compound described in any of (45) to (48) for the manufacture of a laxative.

### Effect of the Invention

The present invention provides a therapeutic agent for irritable bowel syndrome which comprises, as an active ingredient, a compound having an adenosine uptake inhibitory activity, an agent for treating irritable bowel syndrome which comprises, as an active ingredient, a tricyclic compound or a pharmaceutically acceptable salt thereof, and the like.

### Brief Description of the Drawings

[Fig.1]

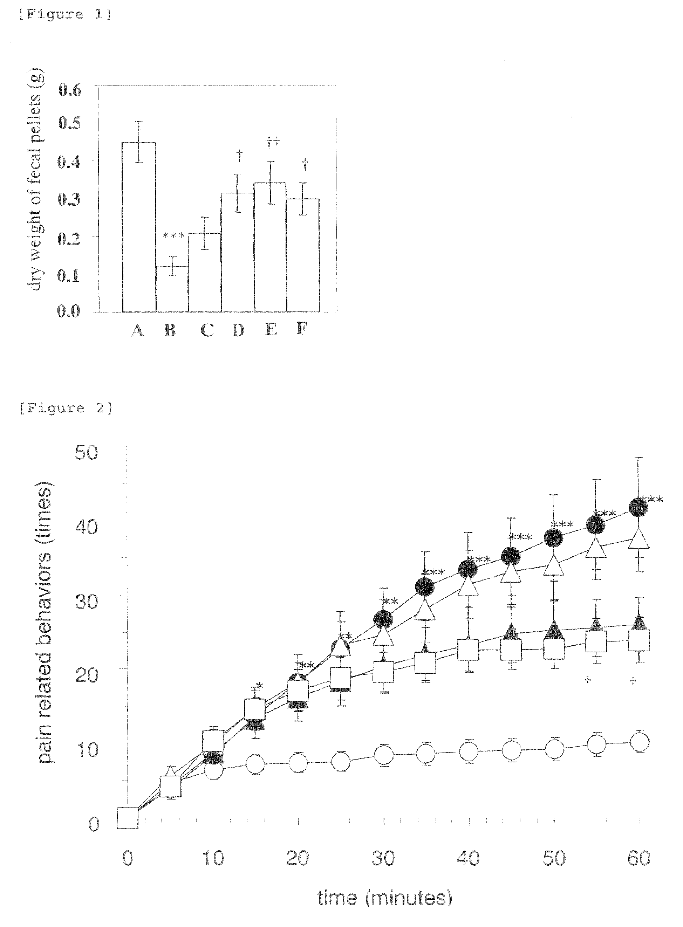

   Fig. 1 shows a fecal amount (g) 0 to 8 hours after the administration of loperamide in each administered group in Test Example 3. The longitudinal axis shows dry weight of fecal pellets (g). A: Normal group
   B: Control group
   C: 0.001 mg/kg of Compound 1-1 administered group
   D: 0.01 mg/kg of Compound 1-1 administered group
   E: 0.1 mg/kg of Compound 1-1 administered group
   F: 1 mg/kg of Compound 1-1 administered group
   ***: p < 0.001 vs. normal group
   †: P < 0.05 vs. control group
   ††: P < 0.01 vs. control group
[Fig. 2]
   Fig. 2 shows numbers of pain-related behaviors in each administered group in Test Example 4. The longitudinal axis shows numbers of pain-related behaviors (times) and abscissa axis shows the time (minutes).
   White circle: Normal group
   Black circle: Control group
   White triangle: 3 mg/kg of Compound 1-1 administered group
   Black triangle: 10 mg/kg of Compound 1-1 administered group
   White square: 30 mg/kg of Compound 1-1 administered group,
   *: p < 0.05 vs. Normal group
   **: P < 0.01 vs. Normal group
   ***: p <0.001 vs. Normal group
   †: P < 0.05 vs. control group

### Best_Mode_for Carrying_Out_the Invention

Hereinafter, compounds represented by Formula (I) are referred to as Compound (I). The same shall apply to the compounds of the other formula numbers.

In the definitions of the groups in Formula (I), the lower alkyl includes, for example, straight-chain or branched alkyl having 1 to 8 carbon atoms, more specifically, methyl, ethyl, propyl, isopropyl, butyl, isobutyl, sec-butyl, tert-butyl, pentyl, hexyl, 1,2,2-trimethylpropyl, heptyl, octyl, etc.

The halogen means fluorine, chlorine, bromine and iodine atoms.

The lower alkyl moiety of the lower alkoxy, mono(lower alkyl)-substituted amino and di(lower alkyl)-substituted amino has the same meaning as the lower alkyl defined above.

The lower alkanoyl of the (lower alkanoyl)amino includes, for example, alkanoyl having 1 to 6 carbon atoms, more specifically, formyl, acetyl, propanoyl, butanoyl, pentanoyl, 2,2-dimethylpropanoyl, hexanoyl, etc.

The lower alkenyl includes, for example, straight-chain or branched alkenyl having 2 to 6 carbon atoms, more specifically, vinyl, allyl, 1-propenyl, methacryl, 1-butenyl, crotyl, pentenyl, hexenyl, etc.

The aryl and the aryl moiety of the arylamino include, for example, phenyl, naphthyl, etc., and the heteroaryl includes, for example, pyridyl, furyl, thienyl, quinolyl, imidazolyl, benzimidazolyl, thiazolyl, etc.

The aralkyl moiety of the aralkylamino includes, for example, aralkyl having 7 to 12 carbon atoms, more specifically, benzyl, phenethyl, naphthylmethyl, etc.

The heterocyclic group includes, for example, heteroalicyclic groups, nitrogen-containing heterocyclic groups, etc. The heteroalicyclic group includes, for example, tetrahydrofuryl, tetrahydrothienyl, chromanyl, etc. The nitrogen-containing heterocyclic group includes, for example, heterocyclic groups containing 1 or 2 nitrogen atoms in the ring and optionally containing other hetero atoms such as oxygen, sulfur, etc. and more specifically, it includes pyrrolidinyl, pipecolinyl, piperazinyl, piperidyl, morpholinyl, thiomorpholinyl, oxazolyl, etc.

The substituted lower alkyl, the substituted lower alkoxy, the mono(substituted lower alkyl)-substituted amino, the di(substituted lower alkyl)-substituted amino, the substituted (lower alkanoyl)amino and the substituted lower alkenyl each have substituents of 1 to a substitutable number (preferably 1 to 6, more preferably 1 to 4) which substituents are the same or different. Examples of the substituents include halogen, hydroxy, nitro, amino, mono(lower alkyl)-substituted amino, di(lower alkyl)-substituted amino, cycloalkyl, substituted cycloalkyl [the substituted cycloalkyl has 1 to 3 substituents which are the same or different, such as halogen, hydroxy, nitro, amino, mono(lower alkyl)-substituted amino, di(lower alkyl)-substituted amino, lower alkoxy, etc.], aryl, substituted aryl (the substituent in the substituted aryl has the same meaning as that in the substituted aryl defined below), aralkyl, substituted aralkyl (the substituent in the substituted aralkyl has the same meaning as that in the substituted aralkyl defined below), lower alkoxy, substituted lower alkoxy [the substituted lower alkoxy 1 to 3 substituents which are the same or different, such as halogen, hydroxy, nitro, amino, mono(lower alkyl)-substituted amino, di(lower alkyl)-substituted amino, lower alkoxy, etc.], lower alkoxycarbonyl, substituted lower alkoxycarbonyl [the substituted lower alkoxycarbonyl has 1 to 3 substituents which are the same or different, such as halogen, hydroxy, nitro, amino, mono(lower alkyl)-substituted amino, di(lower alkyl)-substituted amino, lower alkoxy, etc.] etc. Further, the two substituents on the same carbon atom of the substituted lower alkyl may form, together with the said carbon atom, an aliphatic ring. When the substituted lower alkyl is substituted methyl or substituted ethyl, the number of the substituents in the substituted methyl or the substituted ethyl may be, for example, 1 to 3 and the substituents may be the same or different. For example, the substituents include lower alkyl, substituted lower alkyl [the substituted lower alkyl has 1 to 3 substi-tuents which are the same or different, such as halogen, hydroxy, nitro, amino, mono(lower alkyl)-substituted amino, di(lower alkyl)-substituted amino, lower alkoxy, etc.], etc.

Here, halogen has the same meaning as defined above. The lower alkyl, the lower alkyl moiety of the mono(lower alkyl)-substituted amino, di(lower alkyl)-substituted amino, lower alkoxycarbonyl and lower alkoxy has the same meaning as the lower alkyl defined above and aryl has the same meaning as defined above The cycloalkyl and the cycloalkyl moiety of aliphatic rings include, for example, cycloalkyl having 3 to 8 carbon atoms, more specifically, cyclopropyl, cyclobutyl, cyclopentyl, cyclohexyl, cycloheptyl, cyclooctyl, etc. The aralkyl includes, for example, aralkyl having 7 to 12 carbon atoms, more specifically, benzyl, phenethyl, naphthylmethyl, etc.

The substituted aryl, the substituted heteroaryl, the substituted aralkylamino and the substituted arylamino have 1 to 3 substituents which are the same or different, and examples of the substituents include halogen, hydroxy, amino, lower alkyl, etc.

Here, halogen and lower alkyl have the same meanings at defined above, respectively.

The substituted heterocyclic group has 1 to 3 substituents which are the same or different, and examples of the substituents include halogen, hydroxy, lower alkyl, etc.

Here, halogen and the lower alkyl have the same meanings us defined above, respectively.

In the definitions of formula (Ia), (Ib), (Ic) and (Id), the lower alkyl includes, for example, straight-chain or branched alkyl having 1 to 6 carbon atoms, more specifically, methyl, ethyl, propyl, isopropyl, butyl, isobutyl, sec-butyl, tert-butyl, pentyl, hexyl, 1,2,2-trimethylpropyl, etc.

The halogen means fluorine, chloride, bromine and iodine atoms.

The lower alkyl moiety of the lower alkoxy, mono(lower alkyl)-substituted amino and di(lower alkyl)-substituted amino has the same meaning as that of the lower alkyl defined above.

The lower alkenyl includes, for example, straight-chain or branched alkenyl having 2 to 6 carbon atoms, more specifically, vinyl, allyl, 1-propenyl, methacryl, 1-butenyl, crotyl, pentenyl, hexenyl, etc.

The aryl and the aryl moiety of the arylamino include, for example, phenyl, naphthyl, etc. and the heteroaryl includes, for example, pyridyl, fury, thienyl, quinolyl, imidazolyl, benzimidazolyl, thiazolyl, etc.

The aralkyl and the aralkyl moiety of the aralkylamino include, for example, aralkyl having 7 to 12 carbon atoms, more specifically, benzyl, phenethyl, naphthylmethyl, etc.

The heteroalicyclic group includes, for example, tetrahydrofuryl, tetrahydrothienyl, chromanyl, etc. The nitrogen-containing heterocyclic group includes, for example, a heterocyclic group containing 1 or 2 nitrogen atoms in the ring and optionally containing other hetero atoms such as oxygen , sulfur, etc. in which the nitrogen atom in the ring bonds to the adjacent carbonyl group. For example, it includes pyrrolidinyl, pipecolinyl, piperazinyl, piperidyl, morpholinyl, thiomorpholinyl, oxazolyl, etc.

The cycloalkyl includes, for Example, cycloalkyl having 3 to 8 carbon atoms, more specifically, cyclopropyl, cyclobutyl, cyclopentyl, cyclohexyl, cycloheptyl, cyclooctyl, etc.

The substituted lower alkyl, the substituted lower alkoxy, the mono(substituted lower alkyl)-substituted amino, the di(substituted lower alkyl)-substituted amino, the substituted lower alkenyl and the substituted cycloalkyl have 1 to 3 substituents which are the same or different. Examples of the substituents include halogen, hydroxy, nitro, amino, mono(lower alkyl)-substituted amino, di(lower alkyl)-substituted amino, lower alkoxy, substituted lower alkoxy [the substituted lower alkoxy has 1 to 3 substituents which are the same or different, such as halogen, hydroxy, nitro, amino, mono(lower alkyl)-substituted amino, di(lower alkyl)-substituted amino, lower alkoxy, etc.], lower alkoxycarbonyl, substituted lower alkoxycarbonyl [the substituted lower alkoxycarbonyl has 1 to 3 substituents which are the same or different, such as halogen, hydroxy, nitro, amino, mono(lower alkyl)-substituted amino, di(lower alkyl)-substituted amino, lower alkoxy, etc.] etc.

When the substituted lower alkyl is substituted methyl or substituted ethyl, the number of the substituents in the substituted methyl or the substituted ethyl may be, for example, 1 to 3 and the substituents may bye the same or different. For example, the substituents include lower alkyl, substituted lower alkyl [the substituted lower lower has 1 to 3 substituents which are the same or different, such as halogen, hydroxy, nitro, amino, mono(lower alkyl)-substituted amino, di(lower alkyl)-substituted amino, lower alkoxy, etc.], cycloalkyl, substituted cycloalkyl [the substituted cycloalkyl has 1 to 3 substituents which are the same or different, such as halogen, hydroxy, nitro, amino, mono(lower alkyl)-substituted amino, di(lower alkyl)-substituted amino, lower alkoxy, etc.], aryl, substituted aryl [the substituted aryl has 1 to 3 substituents which are the same or different, such as halogen, hydroxy, nitro, amino, mono(lower alkyl)-substituted amino, di(lower alkyl)-substituted amino, lower alkoxy, etc.], aralkyl, substituted aralkyl [the substituted aralkyl has 1 to 3 substituents which are the same or different, such as halogen, hydroxy, nitro, amino, mono(lower alkyl)-substituted amino, di(lower alkyl)-substituted amino, alkoxy, etc.], etc. Further, the two substituents on the same carbon atom of the substituted methyl or the substituted ethyl may form, together with the said carbon atom, an aliphatic ring.

Here, halogen, cycloalkyl, aryl and aralkyl have the same meanings as defined above, respectively, The lower alkyl and the lower alkyl moiety of the mono(lower alkyl)-substituted amino, di(lower alkyl)-substituted amino, lower alkoxycarbonyl and lower alkoxy have the same meaning as that of the lower alkyl defined above and the cycloalkyl moiety of an aliphatic ring has the same meaning as that of the cycloalkyl defined above.

The substituted aryl, the substituted heteroaryl, the substituted aralkyl, the substituted benzyl, the substituted aralkylamino and the substituted arylmmino have 1 to 3 substituents which are the same or different, of the substituents include halogen, hydroxy, amino, lower alkyl, etc.

Here, halogen and lower alkyl have the same meanings as defined above, respectively.

The substituted heteroalicyclic group and the substituted nitrogen-containing heterocyclic group have 1 to 3 substituents which are the same or different, Examples of the substituents include halogen, hydroxy, lower alkyl, etc.

Here, halogen and lower, alkyl have the same meanings as defined above, respectively.

The pharmaceutically acceptable salts of Compounds (I), (Ia), (Ib), (Ic) and (Id) include pharmaceutically acceptable acid addition salts, for example, inorganic acid addition salts such as hydrochloride, hydrobromide, hydroiodide, nitrate, sulfate, phosphate, etc. and organic acid addition salts such as formate, acetate, benzoate, maleate, fumarate, succinate, tartrate, citrate, oxalate, glyoxylate, aspartate, methanesulfonate, ethanesulfonate, benzenesulfonate, etc.

The tricyclic compounds used in the present invention can be produced according to the methods disclosed in the above publications or similar methods, and can be isolated and purified by purification methods conventionally used in synthetic organic chemistry, such as neutralization, filtration, extraction, wishing, drying, concentration, recrystallization, various kinds of chromatography, etc.

When it is desired to obtain a salt of the tricyclic compound used in the present invention, in the case where it is produced in the form of the salt, it can be subjected to purification as such, and where it is produced in the form of a free base, it can be converted into a salt, after being dissolved or suspended in a suitable solvent, by adding an acid thereto.

There may be optical isomers for some of the tricyclic compounds used in the present invention. All possible stereoisomers and mixtures thereof can be used as active ingredients of the agent for treating irritable bowel syndrome or the adenosine uptake inhibitor of the present invention.

The tricyclic compounds or pharmaceutically acceptable salts thereof used in the present invention may exist in the form of adducts with water or various solvents, which can also be used as active ingredients of the agent for treating irritable bowel syndrome or the adenosine uptake inhibitor of the present invention.

The adenosine uptake inhibitor of the present invention is considered to be useful as an agent for treating, for example, ischemic disease (for example, ischemic heart disease, ischemic cerebrovascular disease, spinal cord injury, etc.), imunoreaction accompanied by organ transplant, epilepsy, thrombosis, arrhythmia, insomnia, pain, inflammatory disease (for example, glomerulonephritis, acute pancreatitis, rheumatic arthritis, inflammatory bowel disease, edema, etc.), IBS, optic nerve disorder, diabetes mellitus, viral disease, tumor, hypertension, Reye's syndrome, convulsion, wound, sleep apnea syndrome, brain injury, Parkinson's disease, Alzheimer's disease, amyotrophic lateral sclerosis, Huntington disease, etc., potentiator of anticancer agent, hair grower, lipolytic agent, etc.

As the compounds having an adenosine uptake inhibitory activity, for example, Dilazep, Draflazine, KF24345, Dipyridamole, S6-(4-nitrobenzyl)mercaptopurine riboside (Nitrobenzyl thioinosine), Lidoflazine, Mioflazine, Soluflazine, R75231, Nimodipine, Diazepam, Clonazepam, Midazolam, Propentofylline, Cilostazol etc. are known [European Journal of Pharmacology, vol. 495, p. 1 (2004)].

As the tricyclic compounds used in the present invention, compounds described in the Table 1 to 6 are included. In the tables, Me and Et respectively represents methyl and ethyl.

**Table 1**

| | | | |
|---|---|---|---|
| 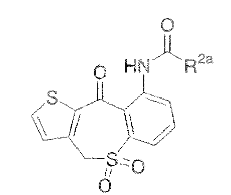 | | | |

| Compound Number | R^{2a} | Compound Number | R^{2a} |
|---|---|---|---|
| 1-1 | 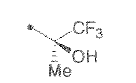 | 1-7 | 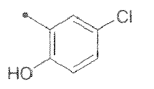 |
| 1-2 | 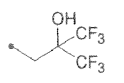 | 1-8 | 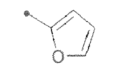 |
| 1-3 | 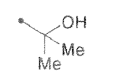 | 1-9 | 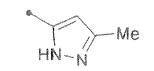 |
| 1-4 | 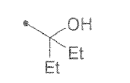 | 1-10 | 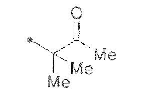 |
| 1-5 | 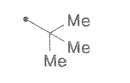 | 1-11 | 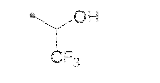 |
| 1-6 | 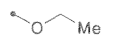 | | |

**Table 2**

| | | | |
|---|---|---|---|
| 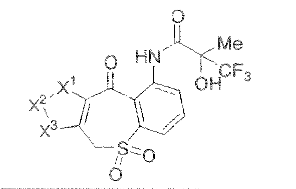 | | | |

| Compound Number | 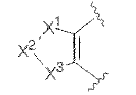 | Compound Number | 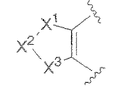 |
|---|---|---|---|
| 2-1 | 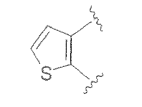 | 2-4 | 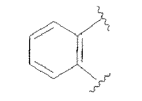 |
| 2-2 | 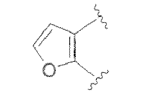 | 2-5 | 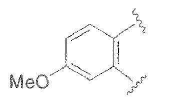 |
| 2-3 | 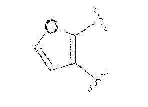 | 2-6 | 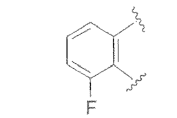 |

**Table 3**

| | |
|---|---|
| 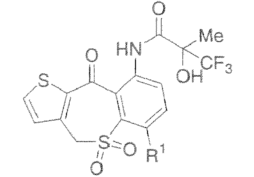 | |

| Compound Number | R¹ |
|---|---|
| 3-1 | F |
| 3-2 | Cl |
| 3-3 | Br |
| 3-4 | Me |

**Table 4**

| Compound Number | Structure |
|---|---|
| 4 | 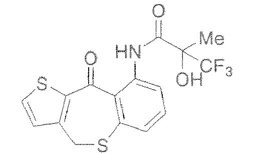 |

**Table 5**

| | | | |
|---|---|---|---|
| 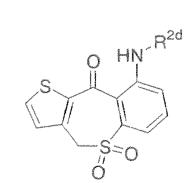 | | | |

| Compound Number | R^{2d} | Compound Number | R^{2d} |
|---|---|---|---|
| 5-1 | 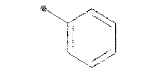 | 5-6 | 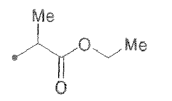 |
| 5-2 | 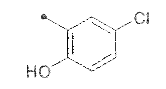 | 5-7 | 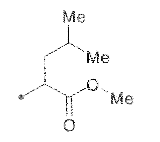 |
| 5-3 | 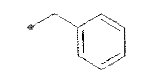 | 5-8 | 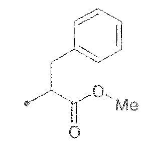 |
| 5-4 | 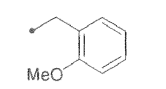 | | |
| 5-5 | 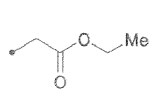 | | |

**Table 6**

| | |
|---|---|
| 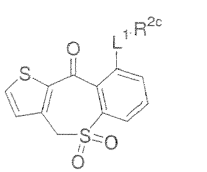 | |

| Compound Number | L¹-R^{2c} |
|---|---|
| 6-1 | 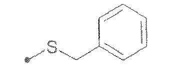 |
| 6-2 | 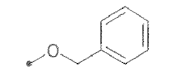 |

Next, the pharmaceutical functions of compounds used in the present invention will be described with preference to test examples.

### Test Example 1: Adenosine uptake inhibitory activity in HEK293 cells

A test was conducted according to the method of Leung et al. [Biochemical Pharmacology, Viol.70, pp.355-362 (2005)]. HEK293 cells (ATCC, Registration No. CRL-1537) were subcultured in a Dulbecco's modified Eagle's medium (manufactured by Invitrogen Corporation) containing 10% of fatal calf serum in a CO₂ incubatoR using a poly-L-lysine-coated 10-cm dish (manufactured by Asahi Techno Glass Corporation). The cultured cells were seeded in a poly-L-lysine-coated 24-well plate (manufactured by Asahi Techno Glass Corporation) and cultured until the cells become confluent. Then, the cells in the wells were washed twice witch 0.5 mL of Na⁺-free buffer (140 mmol/L N-methyl-D-glucose, 5 mmol/L HEPES [4-(2-hydroxyethyl)-1-piperazineethanesulfonic acid], 5 mmol/L KH₂PO₄, 11 mmol/L CaCl₂, mmol/L MgCl₂, 10 mmol/L glucose, pH 7.4), and 0.2 mL of a Na⁺-free buffer in which each compound was dissolved so that the final concentration was each of 50 nmol/L and 500 nmol/L was added to each well (n = 2). After incubation at room temperature for 15 minuted, 0.2 mL of a Na⁺-free buffer containing 100 nmol/L of [³H] adenosine (manufactured by GE Health Care Bioscience Corporation) and each compound at of 50 nmol/L and 500 nmol/L was added to each well. After 1 minute, the reaction solution was removed with an aspirator, and the wells were washed twice with 0.5 mL of ice-cooled PBS (phosphate-buffered saline). Then, 0.5 mL of PBS containing 5% Triton X-100 was added to each well, and the cells were scraped with a scraper. The solution in each well was homogenized by passing through a 20G injection needle ten times and transferred to a scintillation vial containing 4 mL of clean sol (manufacture by Nacalai Tesque Inc.). Further, the wells were washed with 0.5 mL of PBS containing 5% Triton X-100, and the washing solution was added to the same vial. After sufficient agitation, radioactivity was measured using a liquid scintillation counter (Aloka Co., Ltd., LSC-3500). A relative value to 100% of an uptake of a control was indicated by percentage.

The results are shown in Tables 7-1 and 7-2.

### [Table 7-1]

**Table 7-1**

| Test Compound | Uptake (%) | |
|---|---|---|
| | 50 nmol/L | 500 nmol/L |
| 1-1 | 68 | 29 |
| 1-2 | 39 | 17 |
| 1-4 | 73 | 34 |
| 1-5 | 90 | 55 |
| 1-6 | 89 | 68 |
| 1-7 | 74 | 35 |
| 1-8 | 79 | 44 |
| 1-9 | 62 | 26 |
| 1-10 | 94 | 63 |
| 1-11 | 82 | 46 |
| 2-1 | 82 | 49 |
| 2-2 | 86 | 56 |
| 2-4 | 72 | 34 |
| 2-5 | 75 | 44 |
| 3-1 | 96 | 63 |
| 3-4 | 71 | 41 |

### [Table 7-2]

**Table 7-2**

| Test Compound | Uptake (%) | |
|---|---|---|
| | 50 nmol/L | 500 nmol/L |
| 5-1 | 91 | 60 |
| 5-2 | 80 | 44 |
| 5-3 | 63 | 30 |
| 5-4 | 72 | 42 |
| 5-5 | 98 | 68 |
| 5-6 | 80 | 50 |
| 5-7 | 67 | 30 |
| 5-8 | 92 | 69 |
| 6-1 | 75 | 39 |
| 6-2 | 94 | 73 |

These results indicate that Compound (I) has an adenosine uptake inhibitory activity.

Table 8 below indicates that adenosine uptake in HEK293 cells is almost completely inhabited by nitrobenzylthioinosine at a concentration of 100 nmol/L at which only ENT-1 is inhabited [The Journal of Biological Chemistry, Vol.275, No.12, pp.8375-8381 (2000)]. Therefore, it is thought that the adenosine uptake inhibitory activity of Compound (I) is mainly due to the ENT-1 inhibition.

### [Table 8]

**Table 8**

| Test Compound | Uptake (%) | |
|---|---|---|
| | 100 nmol/L | 500 nmol/L |
| Nitrobenzylthioinosine | 8 | 4 |

### Test Example 2: Action on restraint stress-induced defecation

An experiment was carried out according to the method of Kishibayashi et al. [Japanese Journal of Pharmacology, Vol.69, No.4, pp.495-502 (1993)].

Is the experiment, male Wistar rats waged 6 to 7 weeks (supplied from Charles River Laboratories Japan, Inc.) were used. Five to seven rats were housed in each metal cage in a breeding room at a room temperature of 19°C to 25°C and a humidity of 30% to 70% under illumination for 12 hours a day (7 am to 7 pm) and bred by free intake of commercial solid chow and water.

The rats were lightly anesthetized with diethyl ether, and the thoracic trunk and the forelimbs were wrapped with a tape to produce mild stress. One hour after stress initiation, the wet weight of fecal pellets was measured. Each drug solution was orally administered 1 hour before restraint stress. A test compound was suspended at a concentration of 2 mg/mL. in an aqueous solution of 0.5 w/v% methyl cellulose 400cP and orally administered at a volume of 5 mL/kg (at a dose of 10 mg/kg). In a control, an aqueous solution of 0.5 w/v% methyl cellulose 400cP was administered. Ten rats were used in each group. The known adenosine uptake inhibitors, Dilazep, Draflazine, KF24345, and Dipyridamole [European Journal of Pharmacology, Vol.495, p.1 (2004)] were orally administered in doses of 300, 100, 10, and 300 mg/kg, respectively. YM060, a serotonin 5-HT₃ receptor antagonist [The Journal of Pharmacology and Experimental Therapeutics, Vol.259, No.2, pp.815-819 (1991), Astellas Pharma. Inc., online, internet (URL: http:/www.astell.as.com/jp/companry/news/2006/pdf/060131.pdf)], which is being applied as a therapeutic agent for irritable bowel syndrome, was administered at a dose of 0.1 mg/kg. The results are shown in Table 9.

### [Table 9-1]

**Table 9-1**

| Test compound | Wet weight of fecal pellets (g) |
|---|---|
| Normal | 0.14 ± 0.07 |
| Control | 1.71 ± 0.25 |

### [Table 9-2]

**Table 9-2**

| Test compound | Wet weight of fecal pellets (g) |
|---|---|
| Control | 1.38 ± 0.12 |
| Compound 1-1 | 0.27 ± 0.06 |
| Compound 2-3 | 0.45 ± 0.14 |
| Compound 2-5 | 0.37 ± 0.19 |

### [Table 9-3]

**Table 9-3**

| Test compound | Wet weight of fecal pellets (g) |
|---|---|
| Control | 1.66 ± 0.19 |
| Compound 1-2 | 0.78 ± 0.25 |
| Compound 1-4 | 0.44 ± 0.19 |
| Compound 1-5 | 0.97 ± 0.16 |

### [Table 9-4]

**Table 9-4**

| Test compound | Wet weight of fecal pellets (g) |
|---|---|
| Control | 1.40 ± 0.07 |
| Compound 1-6 | 0.31 ± 0.06 |
| Compound 1-7 | 0.59 ± 0.12 |
| Compound 1-8 | 0.66 ± 0.12 |

### [Table 9-5]

**Table 9-5**

| Test compound | Wet weight of fecal pellets (g) |
|---|---|
| Control | 1.44 ± 0.18 |
| Compound 1-9 | 0.39 ± 0.18 |
| Compound 2-2 | 0.53 ± 0.12 |
| Compound 2-4 | 0.39 ± 0.16 |

### [Table 9-6]

**Table 9-6**

| Test compound | Wet weight of fecal pellets (g) |
|---|---|
| Control | 1.28 ± 0.07 |
| Compound 2-6 | 0.28 ± 0.06 |
| Compound 3-1 | 0.41 ± 0.16 |
| Compound 3-3 | 0.81 ± 0.22 |

### [Table 9-7]

**Table 9-7**

| Test compound | Wet weight of fecal pellets (g) |
|---|---|
| Control | 1.71 ± 0.25 |
| Compound 3-2 | 0.53 ± 0.14 |
| Compound 3-4 | 0.67 ± 0.19 |
| Normal | 0.14 ± 0.07 |

### [Table 9-8]

**Table 9-8**

| Test compound | Wet weight of fecal pellets (g) |
|---|---|
| Control | 1.35 ± 0.17 |
| Compound 5-1 | 0.36 ± 0.16 |
| Compound 5-2 | 0.46 ± 0.18 |
| Compound 5-3 | 0.41 ± 0.17 |

### [Table 9-9]

**Table 9-9**

| Test compound | Wet weight of fecal pellets (g) |
|---|---|
| Control | 1.51 ± 0.17 |
| Dilazep 300 mg/kg | 0.84 ± 0.17 |

### [Table 9-10]

**Table 9-10**

| Test compound | Wet weight of fecal pellets (g) |
|---|---|
| Control | 1.06 ± 0.11 |
| Draflazine 100 mg/kg | 0.43 ± 0.13 |
| KF24345 10 mg/kg | 0.48 ± 0.14 |

### []

**Table 9-11**

| Test compound | Wet weight of fecal pellets (g) |
|---|---|
| Control | 1.24 ± 0.23 |
| Dipyridamole 300 mg/kg | 0.59 ± 0.15 |
| YM060 0.1 mg/kg | 0.43 ± 0.11 |

The results indicate that Compound (I) improves restraint stress-induced defecation. This suggests that Compound (I) improves the defecation symptoms of diarrhea-type IBS patients.

### Test Example 3: Activity on loperamide-induced constipation

The therapeutic effect of the compounds of the present application on constipation was evaluated by the method described below. Compound 1-1 was used as a test compound.

In IBS patients, the bowel movement is enhanced, and the symptom of spastic constipation accompanied with an abdominal pain is developed. The spastic constipation is known to be induced by administration of opioid including morphine, loperamide, or the like, and the opioid-induced constipation is used as a model of constipation-type IBS [The Japanese Journal of Pharmacology, Vol.86, No.3, pp.281-288 (2001) and Vol.89, No.2, pp.133-141 (2002)].

To Compound 1-1, 0.5 w/v% methyl cellulose 400 (MC, manufactured by Wako Pure Chemical Industries, Ltd.) was added, followed by lightly mixing with a mixer (TTM-1, Shibata Scientific Technology Ltd.). Then, the resultant mixture was subjected to ultrasonic treatment (1 minute) with an ultrasonic cleaner (Sine Sonic UA100, Hitachi Kokusai, Denki Engineering Co., Ltd.), and suspended by re-mixing with the mixer to prepare a suspension at a concentration of 0.2 mg/mL. The resultant suspension was further diluted with 0.5 w/v% MC to prepare a solution at a concentration of each of 0.02, 0.002, and 0.0002 mg/mL.

Distilled water was added to loperamide hydrochloride (hereinafter, referred to as "loperamide"; Sigma-Aldrich), followed by mixing with a magnetic stirrer to prepare a 1 mg/mL solution. The resultant solution was orally abministered in a volume of 5 mL/kg.

A test was conducted using male SD rats (Charles River Laboratories Japan, Inc.). It has been reported that the rats are decreased in the defecation by loperamide [The Japanese Journal of Pharmacology. Vol.89, No.2, pp.133-141 (2002)]. The rats aged 6 weeks were purchased and used at 7 weeks of age after acclimation for 1 week or more. Five to six rats were housed in each metal cage in a breeding room at a room temperature of 19°C to 25°C and a humidity of 30% to 70% under illumination for 12 hours a day (7 am to 7 pm) and bred by free intake of commercial solid feed (F-2, Funabashi Farms Co.,Ltd) and water. In sampling period of stools, each of the rats was individually placed in a metal cage and let freely drink water under fasting. In the test, the rats showing no obvious abnormality and no diarrhea a day before and an the day of the test (no dirt around the anus) were used.

A day before the test, the rat tails were numbered for identification and the body weights were measured. At the same time, the dirt around the anus and the stool form were observed. In the morning of the day of the test, the rats were orally administered with 0.5 w/v% MC or compound 1-1. One hour after the administration, the rats were orally administered with loperamide. In a normal group rats were orally administered with distilled water. Anyone of the administration solution was administered by oral gavage using an syringe and a tube. After loperamide or distilled water was administered, the rats were moved to the individual breeding cages, and stool sampling was started. Eight hours after the administration of loperamide or distilled water, stools were collected. The collected stools were dried (100°C, 6 hours) with a dryer (SH42, Yamato Scientific Co., Ltd.), and the weight was measured with an electronic balance (AB54, Mettler-Toledo AG).

The groups (in each group, n = 15) comprise as follows:
Normal group: 0.5 w/v% MC 5 mL/kg + distilled water 5 mL/kg
Control group: 0.5 w/v% MC 5 mL/kg + loperamide 5 mg/kg
Compound 1-1 0.001 mg/kg group: Compound 1-1 0.001 mg/kg + loperamide 5 mg/kg
Compound 1-1 0.01 mg/kg group: Compound 1-1 0.01 mg/kg + loperamide 5 mg/kg
Compound 1-1 0.1 mg/kg group: Compound 1-1 0.1 mg/kg + loperamide 5 mg/kg
Compound 1-1 1 mg/kg group: compound 1-1 1 mg/kg + loperamide 5 mg/kg

With respect to the fecal amount, a dry weight was used, and an amount of stools for 0 to 8 hours was used as a measurement parameter. The fecal pellets were collected in a tare (aluminum foil) whose weight was previously measured, and the weight was calculated by subtracting the weight of the tare from the weight including the tare weight.

Statistical analysis was performed using a statistical analysis software SAS (Release 9.1.3, SAS Institute Inc.). Comparison between the fecal amount of the normal group and that of the control group was performed by the F test. When no significant difference was observed in the F test, Student's t-test was conducted, while when a significant difference was observed in the F test, the Aspin-Welch test was conducted.

Comparison between the control group and the compound 1-1. administration group was performed by Bartlett test. When no significant difference was observed in the Bartlett test, a significant difference was confirmed by one-way analysis of variance, and then Dunnett test was conducted. When a significant difference was observed in the Bartlett test, Kruskal-Wallis test was conducted. P < 0.05 was considered as significant difference.

Fig. 1 shows the fecal amount for 0 to 8 hours after the administration of loperamide. The fecal amount of the control group was significantly smaller than that of the normal group.

Compound 1-1 significantly inhibits a decrease in fecal amount after the administration of loperamide in doses of 0.01, 0.1 and 1 mg/kg.

These results demonstrate that Compound 1-1 suppresses loperamide-induced constipation. This suggests that Compound 1-1 improves the stool symptoms of a constipation-type IBS patient.

### Test Example 4: Action on capsaicin-induced visceral pain model

It has been reported that a visceral pain is induced by the administration of capsaicin into the colon [Pain, Vol.92, No.3, pp.335-342 (2001)].

A suspension of Compound 1-1 was prepared with an agate mortar using 0.5 w/v% MC.

Capsaicin (Sigma-Aldrich) was dissolved in a physiological saline solution containing 10% ethanol (Kanto Chemical co., Led.) and 10% polyoxyethylene (20) sorbitan monooleate (Tween 80, Wako Pure Chemical Industries, Ltd.), and the resultant solution was used. A test compound was orally administered in a volume of 10 mL/kg, and MC was administered to a control group in the same volume.

Male ddY mice (Japan SLC, Inc.) with a body weight of 27g to 29g were purchased and used in an experiment. After inspection and acclimation for 2 days or more, the mice showing favorable increases in body weight and no obvious abnormality were used. The animals were housed in a breeding room at a room temperature of 19°C to 25°C and a humidity of 30% to 70% under illumiation for 12 hours a day (7 am to 7 pm) and bred by free intake of commercial solid feed (FR-2, Funabashi Farm Co., Ltd.) and water.

After the oral administration of Compound 1-1 or 0.5 w/v% MC, each mouse was housed in a cage and left therein. Thirty minutes after, 50 µl of 0.1% capsaicin or a vehicle as admistered into the colon of each mouse at a position of 4 cm from the anus. Then, each of the mice was placed in an acrylic cage (8 cm in length × 8 cm in width × 15 cm in height) and recorded with a video (Sony) for 1 hour. After the recording, the following 4 behaviors and symptoms were measured as indexes of capsaicin-induced visceral pain at an interval of 5 minutes. The cumulative results for 60 minutes summarized on a graph.
(1) Licking the abdomen
(2) Stretching
(3) Compressing of the abdomen on a floor
(4) Strong abdominal contraction

Ten mice were used in each group, and the results were shown by average ± standard error. Statistical analysis was performed using a statistical analysis software SAS (Release 8.2, SAS Institute Inc., Cary, NC, USA). Comparison between the control group and the compound administration group was performed by analysis of variance for nested model for confirming a significant difference, followed by the Bartlett test for testing a difference in variances. In the case of equal variances, one-way analysis of variance was performed, and when a significant difference was observed, the Dunnett test was performed. When the variances were not equal, the Kruskal-Wallis test was performed, and when a significant difference was observed, the Steel test was performed. When P < 0.05, it was considered as significantly different.

The results are shown in Fig. 2. In the control group in which 50 µl of 0.1% capsaicin was administered, into the colon, pain-related behaviors were increased as compared with the vehicle administration group (normal group). Compound 1-1 suppressed the pain-related behaviors in doses of 3 and 30 mg/kg, p.o.

These results indicate that Compound 1-1 is effective for a capsaicin-induced visceral pain. This suggests that Compound (I) is effective for treating the abdominal pain of IBS patients.

Test Example 1 indicates that Compound (I) has an adenosine uptake inhibitory activity. And Test Example 2 indicates that Compound (I) has the action of decreasing stress-induced defecation and Test Example 3 indicates that Compound (I) has the action of suppressing constipation. Further, Test Example 4 indicates that Compound (I) is effective for treating abdominal pain. These results show that Compound (I) is useful as a therapeutic agent for IBS. Further, it is shown that a compound having an adenosine uptake inhibitory activity is useful as a therapeutic agent for irritable bowel syndrome like the 5-HT₃ receptor antagonist.

### Test Example 5: Acute toxicity test

Male dd mice (body weight: 20 ± 1 g) were used in groups each of which consisted of three mice, and a test compound was orally or intraperitoneally administered. On the 7th day after the administration, death conditions were observed, and the minimum lethal dose (MLD) was determined.

As a result, the minimum lethal dose of the test compound was 1000 mg/kg in oral administration.

Compound (I) or a pharmaceutically acceptable salt thereof can be used as it is or in any one of various pharmaceutical forms. A pharmaceutical composition of the present invention can be produced by uniformly mixing an effective amount of Compound (I) or its pharmaceutically acceptable salt as an active ingredient with a pharmaceutically acceptable carrier. The pharmaceutical composition is preferably in a unit dosage form suitable for oral or parenteral (including intravenous) administration.

In preparation of the composition in an oral administration form, a useful pharmaceutically acceptable carrier can be used. For example, an oral liquid preparation such as a suspension or a syrup can be produced using water; a saccharide such as sucrose, sorbitol, or fructose; glycol such as polyethylene glycol or propylene glycol; oil such as sesame oil, olive oil, or soybean oil; a preservative such as p-hydroxybenzoate; and a flavor such as strawberry flavor or peppermint. A capsule, a tablet, a powder, and a granule can be produced using an excipient such as lactose, glucose, sucrase, or mannitol; a disintegrator such as starch or sodium alginate; a lubricant such as magnesium stearate or talc; a binder such as polyvinyl alcohol, hydroxypropyl cellulose, or gelatin; a surfactant such as a fatty acid ester; and a plasticizer such as glycerin. A tablet and capsule are the most useful unit oral administration agents because they can easily be administered. In order to produce a tablet and capsule, a solid pharmaceutical carrier is used.

An injection can be prepared using a carrier containing, for example, distilled water, a salt solution, a glucose solution or a mixture of saline and a glucose solution. In this case, an injection is prepared as a solution, a suspension, or a dispersion according to a usual method using an appropriate auxiliary.

Compound (I) or its pharmaceutically acceptable salt can be administered orally, or parenterally as an injection in any one of the above pharmaceutical forms. Although the effective dose and the number of times of administration depend on the administration form and the age, body weight, and symptoms of a patient, the appropriate dose is 1 to 900 mg/60 kg/day and preferably 1 to 200 mg/60 kg/day.

An embodiment of the present invention will be described with reference to reference examples and examples, but the present invention is not limited to these examples.

The compounds below are the same as those described in WO98/46587, and the compounds were synthesized according to the methods described in WO98/46587.
Compound 1-1: Compound 1-25 described in WO98/46587
compound 1-2: compound 3-19 described in WO98/46587
Compound 1-3: Compound 3-12 described in WO98/46587
Compound 1-5: Compound 2-1 described in WO98/46587
Compound 1-6: Compound 3-6 described in WO98/46587
compound 1-7: compound 3-24 described in WO98/46587
compound 1-8: Compound 3-22 described in WO98/46587
Compound 2-1: Compound 1-5 described in WO98/46587
compound 2-2: Compound 1-7 described in WO98/46587
Compound 2-3: Compound 1-11 described in WO98/46587 compound 2-4: Compound 1-3 described in WO98/46587
Compound 2-5: Compound 1-24 described in WO98/46587
compound 2-6: compound 1-19 described in WO98/46587
Compound 3-1: Compound 1-35 described in WO98/46587
compound 3-2: Compound 1-33 described in WO98/46587
Compound 3-4: Compound 1-29 described in WO98/46587
Compound 4 was synthesized according to Reference Example 14 or 15 of WO98/46587.

### Reference Example 1: 5,5-Dioxo-9-(2-ethyl-2-hydroxybutanoylamino)4,10-dihydrothieno[3,2-c][1]benzothiepin-10-one (Compound 1-4)

### Step 1: 9-(Tert-butoxycarbonylamino)-4,10-dihydrothieno[3,2-c][1]benzothiepin-1-one

Di-tert-butyldicarbonate (35.3 g) was added to a solution of 9-amino-4,10-dihydrothieno[3,2-c][1]benzothiepin-10-one (WO98/46587) (20 g) in tetrahydrofuran (THF) (40 mL), followed by stirring under reflux for about 16 hours. Di-tert-butyldicarbonate (17.7 g) was added again, and the resultant mixture was stirred under reflux for about 24 hours. Then, the reaction solution was cooled to room temperature, and silica gel was added to the solution. The solution was concentrated under a reduced pressure, and the residue was purified by silica gel chromatography (hexane/ethyl acetate = 10/1). Then, hexane was added to the resultant crude crystal, and the mixture was stirred at room temperature. The crystal was filtered off, washed with hexane, and then dried under a reduced pressure to obtain the title compound (26.75 g).

### Step 2: 9-(tert-Butoxycarbonylamino)-5,5-dioxo-4,10-dihydrothieno[3,2-c][1]benzothiepin-10-one

A solution of 9-(tert-butoxycarb.nylami-o)-4,10-dihydrothieno[3,2-c][1]benzothiepin-10-one (26.68 g) obtained in the step 1 in ethyl acetate (534 mL) was cooled to 5°C with ice, and m-chloroperbenzoic acid (66.26 g) was gradually added to the solution while the temperature was kept at 10°C or less. Then, the resultant mixture was stirred for 5 minutes under ice-cooling and then stirred at room temperature for about 24 hours. Next, a 1 mol/L aqueous sodium sulfite solution (940 mL) was added dropwise to the reaction mixture under ice-cooling. After the mixture was stirred for 40 minutes under ice-cooling and stirred at room temperature for about 17 hours, ethyl acetate (300 mL) and a saturated aqueous solution of sodium hydrogen carbonate (300 mL) were added to perform extraction. An aqueous layer was further extracted twice with ethyl acetate (300 mL), and the ethyl acetate solution was added to an organic layer. The mixture was washed with a saturated aqueous solution of sodium hydrogen carbonate, water, and saturated saline in that order, dried over anhydrous sodium sulfate, and then concentrated under a reduced pressure. The residue was dissolved in acetone, and silica gel was added to the resultant solution. After the solvent was removed by distillation, the residue was purified by silica gel chromatography (hexane/ethyl acetate = 2/1). Then, diisopropyl ether was added to the resultant crude crystal, and the mixture was stirred at room temperature. The crystal was filtered off, washed with diisopropyl ether, and then dried under a reduced pressure to obtain the title compound (27.74 g).

### Step 3: 9-Amino-5,5-dioxo-4,10-dihydrothieno[3,2-c][1]benzothiepin-10-one

Trifluoroacetic acid (227 mL) was cooled to -7°C with ice in a nitrogen stream, and 9-(tert-butoxycarbonylamino)-5,5-dioxo-4,10-dihydrothieno[3,2-c][1]benzothiepin-10-one (27.68 g) obtained in the step 2 was gradually added, followed by stirring at -7°C to 0°C for about 3 hours. The reaction mixture was poured into iced water (1 L) and stirred at room temperature, and a 10 mol/L aqueous sodium hydroxide solution was added to adjust the mixture to pH 10. Then, ethyl acetate and acetone were added to perform extraction. An organic layer was washed with water and saturated saline in that order, dried over anhydrous sodium sulfate, and concentrated under a reduced pressure. Next, acetone (590 mL) was added to the resultant crude crystal, and the crystal was dissolved by reflux. Then, water (590 mL) was gradually added to the solution under reflux to precipitate a crystal. After cooling to room temperature, the solution was stirred for 3 hours under ice-cooling, and the crystal was filtered off washed with water, and then dried under a reduced pressure to obtain a crude crystal of the title compound (18.54 g). The crude crystal was recrystallized from acetone to obtain the title compound.

### Step 4: Compound 1-4

2-Ethyl-2-hydroxybutanoic acid (44.1 g, 334 mmol) was dissolved in dimethylacetamide (DMA) (416 mL), and thionyl chloride (22.3 mL, 306 mmol) was added to the resultant solution at -13°C, followed by stirring at the same temperature for 1 hour. At the same temperature, 9-amino-5,5-dioxo-4,10-dihydrothieno[3,2-c][1]benzothiepin-10-one (20.7 g, 74.2 mmol) obtained in the step 3 was added to the resultant solution, followed by stirring overnight at room temperature. An acid chloride prepared as described above was again added to the mixture, followed by stirring overnight at room temperature. Then, water (416 mL) was added dropwise (over 1.5 hours) to the reaction mixture, and the mixture was stirred at 0°C for 2.5 hours. The precipitated solid was filtered off and washed with a 70% aqueous ethanol solution (100 mL) to obtain Compound 1-4 (27.8 g, 95.1%).
¹H-NMR (300 MHz, DMSO-d₆, δ) δ: 0.79 (t, J = 7.3 Hz, 6H), 1.46-1.78 (m, 4H), 7.18 (d, J = 5.0 Hz, 1H), 7.80-7.88 (m, 2H), 8.09 (d, J = 5.0 Hz, 1H), 8.44 (dd, J = 7.2, 2.6 Hz, 1H), 10.6 (br, 1H). ESI-MS m/z 392 (M-H)⁻

### Reference Example 2: 5,5-Dioxo-9-[(5-methylpyrazol-3-ylcarbonyl)amino]-4,10-dihydrothieno[3,2-c][1]benzothiepin-10-one (Compound 1-9)

### Step 1: 5-Metliylpyrazole-3-carboxylic acid

Methyl 5-methylpyrazole-3-carboxylate (1.00 6.49 mmol) was dissolved in methanol (6.5 mL), and a 3.5 mol/L aqueous sodium hydroxide solution (6.5 mL) was added to the resultant solution at room temperature, followed by stirring for 1.5 hours. Then, water was added to the reaction mixture, and the mixture was washed with diethyl ether. After pH was adjusted to 1 by adding 1 mol/L hydrochloric acid, extraction with ethyl acetate was performed. An organic layer was washed with water and saturated saline, dried over anhydrous magnesium sulfate, and then concentrated under a reduced pressure. The resultant white crystal was washed with diethyl ether to obtain the title compound (0.25 g, 31%).

### Step 2: 9-[(5-Methylpyrazol-3-ylcarbonyl)aminol-4,10-dihydrothieno[3,2-c][1]benzothiepin-10-one

5-Methylpyrazole-3-carboxylic acid (0.13 g, 1.03 mmol) obtained in the step 1 was dissolved in DMA (5 mL), and thionyl chloride (0.07 mL, 0.96 mmol) was added to the resultant solution on an ice-methanol bath, followed by stirring for 1 hour. Next, at the same temperature, a solution of 9-amino,-4,10-dihydrothien[3,2-c][1]benzothiepin-10-one (WO98/46587) (120 mg, 0.52 mmol) in DMA was added, followed by stirring at room temperature for 1 four. Then, a saturated aqueous solution of sodium hydrogen carbonate was added to the reaction mixture, and the mixture was stirred. After 1 mol/L hydrochloric acid was added, the reaction mixture was extracted with ethyl acetate. An organic layer was washed with water and saturated saline, dried over magnesium sulfate, and then concentrated under a reduced pressure. The residue was purified by silica gel column chromatography (ethyl acetate/hexane = 2/1) to obtain the title compound (103 mg, 55.6%).

### Step 3: Compound 1-9

9-[(5-Methylpyrazol-3-ylcarbonyl)amino]-4,10-dihydrothieno[3,2-c][1]benzothiepin-10-one (87.8 mg, 0.25 mmol) obtained in the step 2 was dissolved in ethyl acetate (10 mL), and m-chloroperbenzoic acid (170 mg, 0.985 mmol) was added to the resultant solution at room temperature, followed by stirring overnight. Then, an aqueous sodium thiosulfate solution was added to the reaction mixture, and the resultant mixture was stirred at room temperature, followed by extraction with ethyl acetate. An organic layer was washed with a saturated aqueous solution of sodium hydrogen carbonate, water, and saturated saline, dried over anhydrous magnesium sulfate, and then concentrated under a reduced pressure. The residue was purified by silica gel column chromatography (ethyl acetate/hexane = 2/1) to obtain compound 1-9 (51.3 mg, 36.8%).
ESI-MS m/z 386 (M-H)⁻

The following compounds can be synthesized according to the steps 2 ann 3 of Reference Example 2 using respective corresponding carboxylic acids and amines.

5,5-Dioxo-9-(2,2-dimethyl-3-oxobutanoylamino)-4,10-dihydrothieno[3,2-c][1]benzothiepin-10-one (compound 1-10): ESI-MS m/z : 390 (M-H)⁻
5,5-Dioxo-9-(2-hydroxy-3,3,3-trifluoropropanoylamino)-4,10-dihydrothieno[3,2-c][1]benzothiepin-10-one (compound 1-11): ESI-MS m/z 404 (M-H)⁻
6-Bromo-5,5-dioxo-9-(3,3,3-trifluoro-2-hydroxy-2-methylpropanoylamino)-4,10-dihydrothieno[3,2-c][1]benzothiepin-10-one (compound 3-3)

### Example 1

### 5,5-Dioxo-9-phenylamino-4,10-dihydrothieno[3,2-c][1]benzothiepin-10-one (Compound. 5-1)

### Step 1: 9-Phenylamino-4,10-dihydrothieno[3,2-c][1]benzothiepin-10-one

9-Amino-4,10-dihydrothieno[3,2-c][1]benzothiepin-10-one (WO98/46587) (200 mg, 0.81 mmol) and phenylboric acid (0.4 g, 3.3 mmol) were dissolved in methylene chloride (5 mL), and copper acetate (0.44 g, 2.4 mmol) and triethylamine (0.5 mL) were added to resultant solution, followed by stirring overnight at room temperature. Copper acetate (0.29 g, 1.6 mmol) and triethylamine (0.23 mL) were again added to the mixture, followed by stirring at room temperature for 4.5 hours. The reaction mixture was concentrated under a reduced pressure, and the residue was purified by silica column chromatography (ethyl acetate/hexane = 1/4) to obtain the title compound (236 mg, 90%).

### Step 2: Compound 5-1

Compound 5-1 was obtained, according to the step 3 of Reference Example 2 using 9-phenylamino-4,10-dihydrothieno[3,2-c][1]benzothiepin-10-one obtained in the step 1.
ESI-MS m/z 356 (M-H)⁻

### Example 2

### 9-(5-Chloro-2-hydroxyphenylamino)-5,5-dioxo-4,1,0-dihydrothieno[3,2-c][1]benzothiepin-10-one (Compound 5-2)

### Step 1: 9-[5-Chloro-2-(methoxylmethyloxy)phenylamino]-4,10-dihydrothieno[3,2-c][1]benzothiepin-10-one

In an argon atmosphere, cesium carbonate (0.73 g, 2.2 mmol), bis(dibenzylideneacetone)plladium (46 mg, 0.080 mmol), 2,2'-bis(diphenylphosphino)-1,1'-binaphthyl (75 mg, 0.12 mmol), 9-amino-4,10-dihydrothieno[3,2-c][1]benzothiepin-10-one (WO98/46587) (474 mg, 1.92 mmol), and 5-chloro-2-(methoxymethyloxy)bromobenzene (400 mg, 1.60 mmol) were added to toluene (6 mL), followed by stirring at 110°C for 4 hours. Cesium carbonate (0.52 g, 1.60 mmol) and 5-chloro-2-(methoxymethyloxy)bromobenzene (400 mg, 1.60 mmol) were to resultant mixture, followed by stirring overnight at 110°C. The reaction mixture was concentrated under a reduced pressure, and the residue was purified by silica gel column chromatography (ethyl acetate/hexane = 1/4) to obtain the title compound (313 mg, 47%).

### Step 2: 9-(5-Chloro-2-hydroxyphenylamino)-4,10-dihydrothieno[3,1-c][1]benzothiepin-10-one

9-[5-Chloro-2-(methoxymethyloxy)phenylamino]-4,10-dihydrothieno[3,2-c][1]benzothlepin-10-one (244 mg, 0.58 mmol) obtained in the step 1 was dissolved in THF (2 mL), any 1 mol/L hydrochloric acid (2 mL) was added to the resultant solution, followed by stirring overnight at 70°C. Then, water was added to the reaction mixture, followed by extraction with ethyl acetate. An organic layer was washed with water, and saturated saline, dried over anhydrous magnesium sulfate, and then concentrated under a reduced pressure. The residue was purified by silica gel column chromatography (ethyl acetate/hexane = 1/4) to obtain the title compound (187 mg, 86.3%).

### Step 3: Compound 5-2

compound 5-2 was obtained according to the step 3 of Reference Example 2 using 9-(5-chloro-2-hydroxyphenylamin-)-4,10-dihydrothieno[3,2-c][1]benzothiepin-10-one obtained in the step 2.
ESI-MS m/z 404 (M-H)⁻

### Example 3

### 9-Benzylamino-5,5-dioxo-4,1,0-dihydrothieno[3,2-c][1]benzothiepin-10-one (Compound 5-3)

### Step 1: 9-Fluoro-5,5-dioxo-4,10-dihydrothieno[3,2-c][1]benzothiepin-10-one

9-Fluoro-4,10-dihydrothieno[3,2-c][1]benzothiepin-10-one (Japanese Unexamined Patent Application Publication No. 2000-053580) (5.0 g, 20.0 mmol) was dissolved in acetonitrile (200 mL) and water (100 mL), and potassium hydrogen monopersulfate double salt (Oxone (registered trade name)) (61.5 g, 100 mmol) was added to the resultant solution, followed by stirring overnight at 60°C. The mixture was cooled to room temperature, and a 1 mol/L aqueous solution of sodium thiosulfate (100 mL) was added to the mixture, followed by stirring at room temperature for 30 minutes. Then, extraction with ethyl acetate was performed, and an organic layer was washed with water and saturated saltine, dried over anhydrous magnesium sulfate, and then concentrated under a reduced pressure. The resultant solid was washed with ethanol to obtain, the title compound (4.43 g, 78%).

### Step 2: Compound 5-3

9-Fluoro-5,5-dioxo-4,10-dihydrothieno[3,2-c][1]benzothiepin-10-one (150 mg, 0.530 mmol) obtained in the step 1 was added to benzylamine (1.5 mL), the resultant mixture was stirred at room temperature for 4.5 hours. Then, 1 mol/L hydrochloric acid was added to the reaction mixture, followed by extraction with ethyl acetate. An organic layer was washed with a saturated aqueous solution of sodium hydrogen carbonate and saturated saltine, dried over anhydrous magnesium sulfate, and then concentrated under a reduced pressure. The residue was purified by silica gel column chromatography (ethyl acetate/hexane = 3/10) to obtain compound 5-3 (174 mg, 88.7%).
ESI-MS m/z 370 (M+H)⁺

Compound 5-4 was obtained according to the step 2 of Reference Example 3 using 2-methoxybenzylamine.
5,5-Dioxo-9-(2-methoxybenzylamino)-4,10-dihydrothieno[3,2-c][1]benzoihiepin-10-one (compounds 5-4): ESI-MS m/z 400 (M+H)⁺

### Example 4

### 5,5-Dioxo.-9-ethoxycarbonylmethylamino-4,10-dihydrothieno[3,2-c][1]benzothiepin-10-one (Compound 5-5)

### Step 1: 9-Ethoxycarbonylmethylamino-4,10-dihydrothieno[3,2-c][1]benzothiepin-10-one

9-Fluoro-4,10-dihydrothieno[3,2-c][1]benzothiepin-10-one (Japanese Unexamined Patent Application Publication No. 2000-053580) (100 mg, 0.39 mmol) was dissolved in 1-methyl-2-pyrrolidinone (3 mL), and glycine ethyl ester hydrochloride (540 mag, 3.87 mmol) and triethylamine (1.1 mL) were added to the resultant solution, followed by stirring at 80°C for 3 hours and at 90°C for 2.5 hours. Then, triethylamine. (1.1 mL) was added to the mixture, and the resultant mixture was stirred overnight at 110°C. Further, glycine ethyl ester hydrochloride (540 mg, 3.87 mmol) and triethylamine (1.1 mL) were added to the mixture, followed by stirring overnight at 110°C. Then, water was added to the reaction mixture, and extraction with ethyl acetate was performed. An organic layer was washed with water and saturated saline, dried over anhydrous magnesium sulfate, and then concentrated under a reduced pressure. The residue was purified by silica gel column chromatography (methyl acetate/hexane = 1/5) to obtain the title compound (63.7 mg, 49.0%).

### Step 2: Compound 5-5

9-Ethoxycarbonylmethylamino-4,10-dihydrothieno[3,2-c][1]benzothiepin-10-one (54.7 mg, 0.16 mmol) obtained in the step 1 was dissolved in acetonitrile (4 mL) and water (2 mL), and Oxone (registered trade name) (0.49 g, 0.80 mmol) was added to the resultant solution, followed by stirring at 50°C for 1 hour. Then, sodium thiosulfate was added to the reaction mixture, followed by extraction with ethyl acetate. An organic layer was washed with water and saturated saline, dried over anhydrous magnesium sulfate, and then concentrated under a reduced pressure. The residue was purified by preparative layer chromatography to obtain compound 5-5 (37.5 mg, 64.1%).
ESI-MS m/z 366 (M+H)⁺

### Example 5

### 5,5-Dioxo-9-[1-(ethoxycarbonyl)ethylamino]-4,10-dihydrothieno[3,2-c][1]benzothiepin-10-one (Compound 5-6)

9-Fluoro-5,5-dioxo-4,11.0-dihydrothieno[3,2-c][1]benzthiepin-10-one (250 mg, 0.890 mmol) obtained in the step 1 of Example 3 was dissolved in 1-methyl-2-pyrrolidinone (8 mL), and DL-alanine ethyl ester hydrochloride (4.0 g, 26 mmol) and triethylamine (5.0 my) were added to the resultant solution, followed by shirring overnight at 50°C. Then, water, and 1 mol/L hydrochloric were added to the reaction mixture, followed by extraction with ethyl acetate. An organic layer was washed with water and saturated saline, dried over anhydrous magnesium sulfate, and then concentrated under a reduced pressure. The residue was purified by silica gel column chromatography (ethyl acetate/hexane = 1/2) to obtain compound 5-6 (159 mg, 51.4%).
ESI-MS m/z 380 (M+H)⁺

The following compounds were synthesized according to Example 5 using respective corresponding amines.
5,5-Dioxo-9-{[1-(methoxycarbonyl)-3-methylbutyl]amino}-4,10-dihydrothieno[3,2-c][1]benzothiepin-10-one (Compound 5-7): ESI-MS m/z 408 (M+H)⁺
5,5-Dioxo-9-{[1-(methoxycarbonyl)-2-phenylethyl]amino}-4,10-dihydrothieno[3,2-c][1]benzothiepin-10-one (compound 5-8): ESI-MS m/z 442 (M+H)⁺

### Example 6

### 9-Benzylthio-5,5-dioxo-4,10-dihydrothieno[3,2-c][1]benzothiepin-10-once (Compound 6-1)

Potassium tert-butoxide (48 mg, 0.43 mmol) was suspended in THF (2.0 mL), and a solution of benzyl, mercaptan (0.05 mL, 0.43 mmol) in THF (1.0 mL) was added dropwise to the resultant suspension under ice cooling, followed by stirring for 30 minutes under ice cooling. Then, a solution of 9-fluoro-5,5-dioxo-4,10-dihydrothieno[3,2-c][1]benzothiepin-10-one (100 mg, 0.35 mmol) in THF (1.0 mL) obtained in the step 1 of Example 3 was added to the mixture, followed by stirring at room temperature for 2 hours. Then, a saturated aqueous solution of ammonium chloride was added the reaction mixture, followed by extraction with ethyl acetate. An organic layer was washed with saturated saline, dried over anhydrous magnesium sulfate, and then concentrated under a reduced pressure. The resultant solid was washed with 2-propanol to obtain compound 6-1 (74 mg, 45%).
ESI-MS *m*/*z* 387 (M+H)⁺; ¹H-NMR (300 MHz, CDCl₃, δ): 4.13 (s, 2H), 4.55 (s, 2H), 6.97 (d, *J* = 4.8 Hz, 1H), 7.17-7.26 (m, 5H), 7.55 (t, *J* = 7.5 Hz, 1H), 7.67 (d, *J* = 4.8 Hz, 1H), 7.76 (d, *J* = 7.5 Hz, 1H), 7.91 (d, *J* = 7.5 Hz, 1H).
ESI-MS *m*/*z* 387 (M+H)⁺

### Example 7

### 9-Benzyloxy-5,5-dioxo-4,10-dihydrothieno[3,2-c][1]benzothiepin-10-one (compound 6-2)

### Step 1: Methyl 2-dimethylthlocarbamoyloxy-6-methoxybenzoate

Methyl 2-hydroxy-6-methoxybenzoate (16.9 g, 89.2 mmol) was dissolved in *N*,*N*-dimethylformamide (DMF) (150 mL), and dimethylthiocarbamoyl chloride (16.3 g, 132 mmol) and 1,4-diazabicyclo[2.2.2]octane (18.5 g, 165 mmol) were added to the resultant solution, followed by sitting overnight at room temperature. Then, water, was added to the mixture, and extraction with ethyl acetate was performed. An organic layer was washed witch water and saturated saline, dried over anhydrous magnesium sulfate, and then concentrated under a reduced pressure. The residue was purified by silica gel column chromatography to obtain the title compound (20.7 g, 86%).

### Step 2: Methyl 2-dimethylcabamoylsulfanyl-6-methoxybenzote

Diphenyl ether (30 mL) was added to methyl 2-dimethylthiocarbamoyloxy-6-methoxybenzoate (20.0 g, 74.3 mmol) obtained in the step 1, and the resultant mixture was stirred at 220°C for 30 minutes. The mixture was purified by silica gel column chromatography to obtain the title compound (20.0 g, quantitative).

### Step 3: Methyl 2-methoxy-6-(thiophen-3-ylmethylsulfanyl)benzoate

Methyl 2-dimethylca.rbamoylsulfanyl-6-methoxybenzoate (20.0 g, 74.3 mmol) obtained in the step 2 was dissolved in methanol (300 mL), and a 28% sodium methoxide/methanol solution (43 g, 223 mmol) was added to the resultant solution, followed by stirring at room temperature for 2 hours. Then, the mixture was cooled in an ice bath, and a solution, of thiophene-3-methyl chloride (15.3 g, 127 mnol) in methanol (30 mL) was added to the mixture, followed by stirring at 0°C for 30 minutes. Then, a saturated aqueous solution, of ammonium chloride was added to the reaction mixture, and the precipitated solid was filtered off and washed with water and n-hexane to obtain the title compound (19.5 g, 89%).

### Step 4: Lithium 2-methoxy-6-(thiophen-3-ylmethylsulfanyl)benzoate

Methyl 2-methoxy-6-(thiophen-3-ylmethylsulfanyl)benzoate (19.5 g, 66.2 mmol) obtained in the step 3 was dissolved in methanol (300 mL), and mol/L lithium hydroxide (306 mL, 306 mmol) was added to the resultant solution, followed by stirring at room temperature to for 48 hours. Then, water was added to the reaction mixture, and the precipitated solid was filtered off to obtain the title compound (18.0 g, 97%).

### Step 5: 9-Methoxy-4,10-dihydrothieno[3,2-c][1]benzothiepin-10-one

Lithium 2-Methoxy-6-(thiophen-3-ylmethylsulfanyl)benzoate (18.0 g, 64.3 mmol) obtained in the stop 4 was suspended in methylene chloride (300 mL), and trifluoroacetic anhydride (10.0 mL, 70.7 mmol) was added to the resultant suspension at 0°C, followed by stirring overnight at temperature. Then, 1 mol/L hydrochloric acid was added to the mixture, and extraction with methylene chloride was performed. An organic layer was washed with water and saturated saline, dried over anhydrous magnesium sulfate, and then concentrated under a reduced pressure. The residue was purified by silica gel column chromatography to obtain the title compound (15.2 g, 90%).

### Step 6: 9-Hydroxy-4,10-dihydrothieno[3,2-c][1]benzothiepin-10-one

Acetic acid (150 and hydrobromic (100 mL) were added to 9-methoxy-4,10-dihydrothieno[3,2-c][1]benzothiepin-10-one (15.2 g, 57.8 mmol) obtained in the step 5, followed by reflux under heating for 24 hours. The reaction mixture was cooled to room temperature and then concentrated under a reduced pressure, and water was added to the residue, followed by extraction with methylene chloride. An organic layer was washed with a saturated aqueous solution of sodium hydrogen carbonate and saturated saline, dried over anhydrous magnesium sulfate, and then concentrated under a reduced pressure. The residue was purified by silica gel column chromatography to obtain the title compound (12.9 g, 90%).

### Step 7: 9-Hydroxy-4,10-dihydrothieno[3,2-c][1]benzothiepine-5,5,10-trione

9-Hydroxy-4,10-dihydrothieno[3,2-c][1]benzothiepin-10-one (1.0 g, 4.03 mmol) obtained in the step 6 was dissolved in acetonitrile (100 mL) and water (50 mL), and Oxone (registered trade name) (9.90 g, 16.1 mmol) was added to the resultant solution followed by stirring at 60°C for 3 hours. Then, a saturated aqueous solution of sodium thiosulfate was added to the reaction mixture, the mixture was stirred at temperature, followed by extraction with ethyl acetate. An organic layer was washed with water saturated saline, dried over anhydrous magnesium sulfate, and concentrated under a reduced pressure. The resultant solid was with acetone to obtain the title compound (0.79 g, 74%).

### Step 8: Compound 6-2

9-Hydroxy-4,10-dihydrothirno[3,2-c][1]benzothiepine-5,5,10-trione (0.1 g, 0.357 mmol) obtained in the stop 7 was dissolved in DMF (2 mL), potassium carbonate (0.1 g, 0.714 mmol) and benzyl bromide (0.047 mL, 0.428 mmol) were added to the resultant solution, followed by stirring overnight at temperature. Then, water was added to the reaction mixture, followed by extraction with ethyl acetate. An organic layer was washed with saturated saline, dried over anhydrous magnesium sulfate, and then concentrated under a reduced pressure. The resultant solid was purified by silica gel column chromatography to obtain Compound 6-2 (0.042 g, 32%).
ESI-MS *mlz* 371 (M+H)⁺; ¹H-NMR (270 MHz, CDCl₃,δ): 4.68 (s, 2H), 5.21 (s, 2H), 7.12 (d, *J* = 5.0 Hz, 1H), 7.23-7.43 (m, 6H), 7.69-7.73 (m, 2H), 8.10 (d, *J* = 7.3 Hz, 1H),
ESI-MS m/z 371 (M+H)⁺

### Example 8

### Formulation Example 1: Tablet

Tablets having the following composition were prepared by a usual method.

First, 250 g of Compound 1-1, 1598.5 g of mannitol, 100 g of carboxylmethyl starch, 10 g of light anhydrous silicic acid, 40 g of magnesium stearate, and 1.5 g of yellow iron sesquioxide were by a usual method. The resultant mixture tableted with a tableting machine (Manufactured by Kikusui Seisakusho Ltd., Purepress Correct-12 model) having a punch die of 8 mm in diameter to prepare tablets (containing 25 mg of an active component tablet). The prescription is in Table 10.

### [Table 10]

**Table 10**

| Prescription | | |
|---|---|---|
| Compound 1-1 | 25 | mg |
| Mannitol | 159.85 | mg |
| Sodium carboxylmethyl starch | 10 | mg |
| Light anhydrous silicic acid | 1 | mg |
| Magnesium stearate | 4 | mg |
| Yellow iron sesquioxide | 0.15 | mg |
| | 200 | mg |

### Example 9

### Formulation Example 2: Injection

Injections having the following composition are prepared by a usual method.

Compound 1-1 (1 g) and 5 g of D-mannitol are added to distilled for injection and mixed, and further hydrochloric acid and an aqueous sodium hydroxide solution are added to the mixture to adjust pH to 6. Then, distilled water is added to a total of 1000 mL. Each glass vial is aseptically filled with 2 mL of the resultant mixture to injections (containing 2 mg of an active component per vial). The prescription is shown in Table 11.

**Table 11**

| Prescription | | |
|---|---|---|
| Compound 1-1 | 2 | mg |
| D-mannitol | 10 | mg |
| Hydrochloric acid | proper | amount |
| Aqueous sodium hydroxide solution | proper | amount |
| Distilled water for injection | proper | amount |
| | 2.00 | mL |

### Industrial Applicability

The present invention provides a therapeutic agent for irritable bowel syndrome comprises, as an active ingredient, a compound an adenosine inhibitory activity, a therapeutic for irritable bowel syndrome which comprises, as an active ingredient, a tricyclic compound or a pharmaceutically acceptable salt thereof, and the like.

## Claims

1. A therapeutic agent for irritable bowel syndrome which comprises, as an active ingredient, a compound having an adenosine inhibitory activity.

2. An antidiarrheal which comprises, as an active ingredient, a compound having an adenosine inhibitory activity.

3. A laxative which comprises, as an active ingredient, a compound having an adenosine uptake inhibitory activity.

4. A therapeutic agent for irritable bowel syndrome which comprises, as an active ingredient, a tricyclic compound represented by Formula (I) 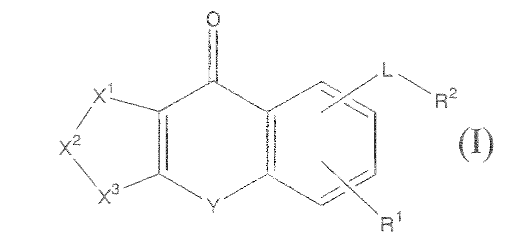 {wherein L represents as oxygen atom, a sulfur atom, -N(R⁹)-(wherein R⁹ represents a hydrogen atom or substituted or unsubstituted lower alkyl), -NHC(=O)- or -C(=O)NH-,
R¹ represents a hydrogen atom, halogen, substituted or unsubstituted alkyl, or substituted or unsubstituted lower
X¹-X²-X³ represents CR⁵=CR⁶-CR⁷=CR⁸ [wherein R⁵, R⁶, R⁷ and R⁸ may be the same or different and each represents a hydrogen atom, halogen, hydroxy, nitro, amino, mono(lower alkyl)-substituted amino, di(lower alkyl)-substituted amino, substituted or unsubstituted lower alkyl, substituted or unsubstituted lower alkoxy, or substituted or unsubstituted (lower alkanoyl)amino], N(O)ₘ=CR⁶-CR⁷=CR⁸ (wherein R⁶, R⁷ and R⁸ have the same meanings as above, respectively and m represent 0 or 1),
CR⁵=CR⁶-N(O)ₘ=CR⁸ (wherein R⁵, R⁶ R⁸ and m have the same meanings as defined above, respectively), CR⁵=CR⁶-CR⁷=N(O)ₘ (wherein R⁵, R⁶, R⁷ and m have the same meanings as defined above, respectively), CR⁵=CR⁶-O (wherein R⁵ and R⁶ have the same meanings as defined above, respectively), CR⁵=CR⁶-S (wherein R⁵ and R⁶ have the same meanings as defined above, respectively), O-CR⁷=CR⁸ (wherein R⁷ and
R⁸ have the same meanings as defined above, respectively),
S-CR⁷=CR⁸ (wherein R⁷ and R⁸ have the same meanings as defined above, respectively) or O-CR⁷=N (whereon R⁷ the meaning as defined above),
Y represents -CH₂S-, -CH₂SO-, -CH₂SO₂-, -CH₂O-, -CH=CH-, -(CH₂)ₚ-(wherein p represents an integer of 0 to 2), -SCH₂-, -SOCH₂-, -SO₂CH₂- or -OCH₂-, and
R² represents a hydrogen atom, amino, substituted or unsubstituted lower alkyl, substituted or unsubstituted lower alkenyl, substituted or unsubstituted lower alkoxy, mono(substituted or unsubstituted lower alkyl)-substituted amino, di(substituted or unsubstituted lower alkyl)-substituted amino, substituted or unsubstituted aryl, substituted or unsubstituted heteroaryl, substituted or unsubstituted aralkylamino, substituted or unsubstituted arylamino, or a substituted or unsubstituted heterocyclic group} or a pharmaceutically acceptable salt thereof.

5. An antidiarrheal which comprises, as an active ingredient, the tricyclic compound or the pharmaceutically acceptable salt thereof described in Claim 4.

6. A laxative which comprises, as an active ingredient, the tricyclic compound or the pharmaceutically acceptable salt thereof described in Claim 4.

7. An adenosine uptake inhibitor which comprises, as an active ingredient, the tricyclic compound or the pharmaceutically acceptable salt thereof in Claim 4.

8. A therapeutic agent for irritable bowel syndrome which comprises, as an active ingredient, a tricyclic compound represented by Formula (Ia) 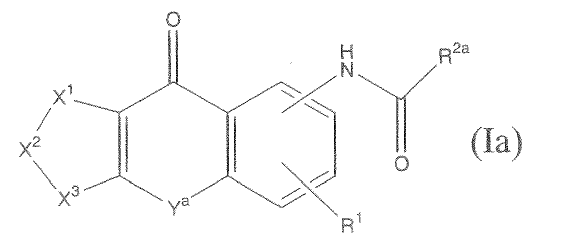 [wherein R¹ and X¹-X²-X³ have the same meanings as defined above, respectively,
Y^{a} represents -CH₂SO₂-, -SCH₂-, -SOCH₂-, -SO₂CH₂- or -OCH₂-, and
when Y^{a} is -CH₂SO₂-, -SCH₂-, -SOCH₂- or -SO₂CH₂-,
R^{2a} represents a hydrogen atom, amino, substituted or unsubstituted lower alkyl, substituted or unsubstituted lower alkenyl, substituted or unsubstituted lower alkoxy, mono(substituted or unsubstituted lower alkyl)-substituted amino, di(substituted or unsubstituted lower alkyl)-substituted amino, substituted or unsubstituted aryl, substituted or unsubstituted heteroaryl, substituted or unsubstituted aralkylamino, substituted or unsubstituted arylamino, a substituted or unsubstituted heteroalicyclic group, or a substituted or unsubstituted nitrogen-containing heterocyclic group, and
when Y^{a} is -OCH₂-,
R^{2a} represents a hydrogen atom, amino, trifluoromethyl, substituted or unsubstituted lower alkenyl, substituted, or unsubstituted lower alkoxy, mono(substituted or unsubstituted lower alkyl)-substituted amino, di(substituted or unsubstituted lower alkyl)-substituted amino, substituted or unsubstituted aryl, substituted or unsubstituted heteroaryl, substituted or unsubstituted aralkylamino, substituted or unsubstituted arylamino, a substituted or unsubstituted heteroalicyclic group, a substituted or unsubstituted nitrogen-containing heterocyclic group, or Formula (II) 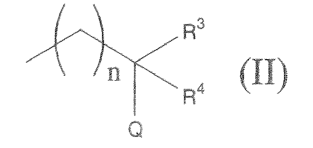 (wherein n is 0 or 1; R³ and R⁴ may be the same or different and each represents a hydrogen atom, substituted or unsubstituted lower alkyl, substituted or unsubstituted cycloalkyl, substituted or unsubstituted aryl, or substituted or unsubstituted aralkyl, or R³ and R⁴ may be combined together with the adjacent carbon atom thereto to form cycloalkyl; and Q represents halogen, amino, hydroxy, or substituted or unsubstituted lower alkoxy)] or a pharmaceutically acceptable salt thereof.

9. The therapeutic agent for irritable bowel syndrome according to Claim 8 wherein Y^{a} is -CH₂SO₂-, -SCH₂-, -SOCH₂- or -SO₂CH₂-.

10. The therapeutic agent for irritable bowel syndrome according to Claims 8 wherein Y^{a} is -OCH₂-.

11. The therapeutic agent for irritable bowel syndrome according to any of Claims 8 to 10 wherein R¹ is a hydrogen atom, halogen or substituted or unsubstituted lower alkoxy.

12. The therapeutic agent for irritable bowel syndrome according to of Claims 8 to 10 wherein R¹ is a hydrogen atom.

13. The therapeutic agent for irritable bowel syndrome according to of Claims 8, 11 and 12 wherein Y^{a} is -CH₂SO₂-, -SO₂CH₂- or -OCH₂-,

14. The therapeutic agent for irritable bowel syndrome according to any of Claims 8, 11 and 12 wherein Y^{a} is -CH₂SO₂- or -SO₂CH₂-.

15. The therapeutic agent for irritable bowel syndrome according to any of Claim 8, 11 and 12 wherein Y^{a} is -CH₂SO₂-.

16. The therapeutic for irritable bowel syndrome according to any of Claims 8 to 15 wherein X¹-X²-X-³ is S-CR⁷=CR⁸ (wherein R⁷ and R⁸ have the same meanings as defined above, respectively).

17. The therapeutic agent for irritable bowel syndrome according to any of Claims 8 to 15 wherein X¹-X²-X³ is CR⁵=CR⁶-CR⁷=CR⁸ (wherein R⁵, R⁶, R⁷ and R⁸ have the same meanings as defined above, respectively).

18. The therapeutic agent for irritable bowel syndrome according to of claims 8 to 17, wherein R^{2a} is Formula (II) 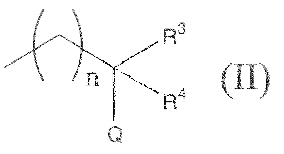 (wherein n, R³, R⁴ and Q have the same meanings as defined above, respectively).

19. The therapeutic agent for irritable bowel syndrome according to Claim 18, wherein n is 0.

20. The therapeutic agent for irritable bowel syndrome according to Claim 19, wherein R³ is methyl, R⁴ is trifluoromethyl, and Q is hydroxy.

21. The therapeutic agent for irritable bowel syndrome according to Claim 8, wherein R¹ is a hydrogen atom, Y^{a} is -CH₂SO₂-, X¹-X²-X³ is S-CR⁷=CR⁸ (wherein R⁷ and R⁸ have the meanings as defined above, respectively), and R^{2a} is Formula (III)

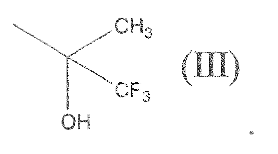

22. An antidiarrheal which comprises, as an active ingredient, the tricyclic compound or the pharmaceutically acceptable salt thereof described in any of Claims 8 to 21.

23. A laxative which comprises, as an active ingredient, the tricyclic compound or the pharmaceutically acceptable salt thereof described in any of Claims 8 to 21.

24. Am adenosine uptake inhibitor which comprises, as an active ingredient, the tricyclic compound or the pharmaceutically acceptable salt thereof described in any of Claims 8 to 21.

25. A therapeutic for irritable bowel syndrome which comprises, as an active ingredient, a tricyclic compound represented by Formula (Ib) 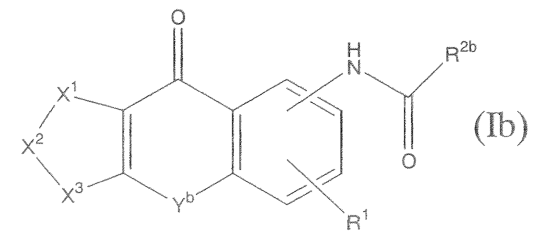 [wherein R¹ and X¹-X²-X³ have the same meanings as defined above, respectively, Y^{b} represents -CH₂O-, -CH₂S-, -CH₂SO-, -CH=CH- or -(CH₂)_{P}- (wherein p the same meaning as above), and R^{2b} represents Formula (III) 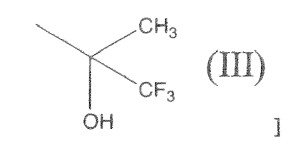 or a pharmaceutically salt thereof.

26. The therapeutic agent for irritable bowel syndrome according to Claim 25, wherein X¹-X²-X³ is CR⁵=CR⁶-CR⁷=CR⁸ (wherein R⁵, R⁶, R⁷ and R⁸ have the same meanings as defined above, respectively) or CR⁵=CR⁶-CR⁷=N (wherein R⁵, R⁶ and R⁷ have the same meanings as above, respectively).

27. The therapeutic agent for irritable bowel syndrome according to Claim 25, wherein X¹-X²-X³ is CR⁵=CR⁶-O (wherein R⁵ and R⁶ have the same meanings as defined above, respectively) or CR⁵=CR⁶-S (wherein R⁵ and R⁶ have the same meanings as defined above, respectively).

28. The therapeutic agent for irritable bowel syndrome according to Claim 25, wherein X¹-X²-X³ is O-CR⁷=CR⁸ (wherein R⁷ and R⁸ have the same meanings as defined above, respectively) or S-CR⁷=CR⁸ (wherein R⁷ and R⁸ have the same meanings as defined above, respectively).

29. The therapeutic agent for irritable bowel syndrome according to any of Claims 25 to 28, wherein Y^{b} is -CH₂O-.

30. The therapeutic agent for irritable bowel syndrome according to any of Claims 25 to 28, wherein Y^{b} is -(CH₂)ₚ-(wherein p has the same as defined above).

31. The therapeutic agent for irritable bowel syndrome according to Claim 30, wherein p is 0.

32. The therapeutic agent for irritable bowel syndrome according to Claim 30, wherein p is 2.

33. The therapeutic agent for irritable bowel syndrome according to any of Claims 25 to 28, wherein Y^{b} is -CH=CH-.

34. The therapeutic agent for irritable bowel syndrome according to any of Claims 25 to 28, wherein Y^{b} is -CH₂S- or -CH₂SO-.

35. An antidiarrheal which comprises, as an active ingredient, the tricyclic compound or the pharmaceutically acceptable salt thereof described in any of Claims 25 to 34.

36. A laxative which comprises, as an active ingredient, the tricyclic compound or the pharmaceutically acceptable salt thereof described in any of Claims 25 to 34.

37. An adenosine uptake inhibitory which comprises, as an active ingredient, the tricyclic compound or the pharmaceutically acceptable salt thereof described in any of Claims 25 to 34.

38. A tricyclic compound represented by Formula (Ic) 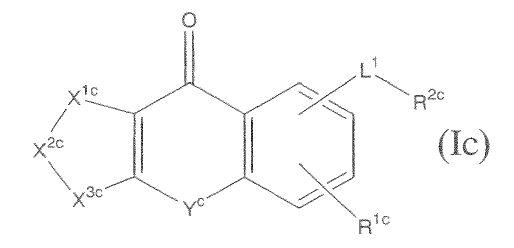 [wherein L¹ represents an oxygen atom or a sulfur atom,
R^{1c} represents a hydrogen atom or substituted or unsubstituted lower alkyl,
X^{1c}-x^{2c}-X^{3c} represents O-CR⁷=CR⁸ (wherein R⁷ and R⁸ have the same meanings as defined above, respectively) or S-CR⁷=CR⁸ (wherein R⁷ and R⁸ have the same meanings as defined above, respectively),
Y^{c} represents -CH₂S-, -CH₂SO- or -CH₂SO₂-, and
R^{2c} represents substituted or unsubstituted lower alkyl] or a pharmaceutically acceptable salt thereof.

39. The tricyclic compound or the pharmaceutically acceptable salt thereof according to Claim 38, wherein R^{1c} is a hydrogen atom, X^{1c}-X^{2c}-X^{3c} is S-CR^{7c}=CR^{8c} (wherein R^{7c} and R^{8c} may be the same or different and each represents a hydrogen atom or substituted or unsubstituted lower alkyl),
Y^{c} is -CH₂SO₂- and
R^{2c} is substituted or unsubstituted benzyl.

40. A pharmaceutical composition which comprises, as an active ingredient, the tricyclic compound or the pharmaceutically acceptable salt thereof described in Claim 38 or 39.

41. A therapeutic agent for irritable bowel syndrome which comprises, as an active ingredient, the tricyclic compound or the pharmaceutically acceptable salt thereof described in Claim 38 or 39.

42. An antidiarrheal which comprises, as an active ingredient, the tricyclic compound or the pharmaceutically acceptable salt thereof, described in Claim 38 or 39.

43. A laxative which comprises, as an active ingredient, the tricyclic compound or the pharmaceutically acceptable salt thereof, described in Claim 38 or 39.

44. An adenosine uptake inhibitor which comprises, as an active ingredient, the tricyclic compound or the pharmaceutically acceptable salt thereof described in Claim 38 or 39.

45. A tricyclic compound represented by Formula (Id) 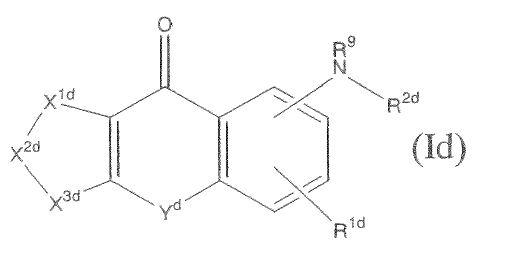 [wherein R⁹ has the same meaning as defined above,
R^{1d} represents a hydrogen atom, or substituted or unsubstituted lower alkyl,
X^{1d}-X^{2d}-X^{3d} represents O-CR⁷=CR⁸ (wherein R⁷ and R⁸ have the same meanings as defined above, respectively) or S-CR⁷=CR⁸ (wherein R⁷ and R⁸ have the same meanings as defined above, respectively),
Y^{d} represents -CH₂S-, -CH₂SO-, or -CH₂SO₂-, and
R^{2d} represents substituted or unsubstituted lower alkyl or substituted or unsubstituted aryl] or a pharmaceutically acceptable salt thereof.

46. The tricyclic compound or the pharmaceutically acceptable salt thereof according to Claim 45, wherein R^{1d} is a hydrogen atom, X^{1d}-X^{2d}-X^{3d} is S-CR^{7d}=CR^{8d} (wherein R^{7d} and R^{8d} may be the same or different and each represents a hydrogen atom or substituted or unsubstituted lower alkyl),
Y^{d} represents -CH₂SO₂- and
R⁹ is a hydrogen atom.

47. The tricyclic compound or the pharmaceutically acceptable salt thereof according to Claim 46, wherein R^{2d} is substituted or unsubstituted lower alkyl.

48. The tricyclic compound or the pharmaceutically acceptable salt thereof according to Claim 46, wherein R^{2d} is substituted or unsubstituted aryl.

49. A pharmaceutical composition which comprises, as an active ingredient, the tricyclic compound or the pharmaceutically acceptable salt thereof described in any of Claims 45 to 48.

50. A therapeutic agent for irritable bowel syndrome which comprises, as an active ingredient, the tricyclic compound or the pharmaceutically acceptable salt thereof described in any of Claims 45 to 48.

51. An antidiarrheal which comprises, as an active ingredient, the tricyclic compound or the pharmaceutically acceptable salt thereof described in any of Claims 45 to 48.

52. A laxative which comprises, as an active ingredient, the tricyclic compound or the pharmaceutically acceptable salt thereof described in any of Claims 45 to 48.

53. An adenosine uptake inhibitory which comprises, as an active ingredient, the tricyclic compound or the pharmaceutically acceptable salt thereof described in any of Claims 45 to 48.

54. A method for treating irritable bowel syndrome which comprises a step of administering an effective amount of a compound having an adenosine uptake inhibitory activity.

55. A method for treating diarrhea which comprises a step of administering an effective amount of a compound having an adenosine uptake inhibitory activity.

56. A method for treating constipation which comprises a step of administering an effective amount of a compound having an adenosine uptake inhibitory activity.

57. A method for treating irritable bowel syndrome which comprises a step of administering an effective amount of the tricyclic compound described in any of Claims 4, 8 to 21 and 25 to 34.

58. A method for treating diarrhea which comprises a step of administering an effective amount of the tricyclic compound described in any of Claims 4, 8 to 21 and 25 to 34.

59. A method for treating constipation which comprises a step of administering an effective amount of the tricyclic compound described in any of Claims 4, 8 to 21 and 25 to 34.

60. A method for treating irritable bowel syndrome which comprises a step of administering an effective amount of the tricyclic compound described in Claim 38 or 39.

61. A method for treating diarrhea which comprises a step of administering an effective amount of the tricyclic compound described in Claim 38 or 39.

62. A method for treating constipation which comprises a step of administering an effective amount of the tricyclic compound described in Claim 38 or 39.

63. A method for treating irritable bower syndrome which comprises a step of administering an effective amount of the tricyclic compound described in any of Claims 45 to 48.

64. A method for treating diarrhea which comprises a step of administering an effective amount of the tricyclic compound described in any of Claims 45 to 48.

65. A method for treating constipation which comprises a step of administering an effective amount of the tricyclic compound described in any of Claims 45 to 48.

66. Use of a compound having an adenosine uptake inhibitory activity for the manufacture of a therapeutic agent for irritable bowel syndrome.

67. Use of a compound having an adenosine uptake inhibitory activity for the manufacture of an antidiarrheal.

68. Use of a compound having an adenosine uptake inhibitory activity for the manufacture of a laxative.

69. Use of the tricyclic compound described in any of Claims 4, 8 to 21 and 25 to 34 for the manufacture of a therapeutic agent for irritable bowel syndrome.

70. Use of the tricyclic compound described in any of Claims 4, 8 to 21 and 25 to 34 for the manufacture of an antidiarrheal.

71. Use of the tricyclic compound described in any of Claims 4, 8 to 21 and 25 to 34 for the manufacture of a laxative.

72. Use of the tricyclic compound described in Claim 38 or 39 for the manufacture of a therapeutic agent for irritable bowel syndrome.

73. Use of the tricyclic compound described in Claim 38 or 239 for the manufacture of an antidiarrheal.

74. Use of the tricyclic compound described in Claim 38 or 39 for the manufacture of a laxative.

75. Use of the tricyclic compound described in any of Claims 45 to 48 for the manufacture of a therapeutic agent for irritable bowel syndrome.

76. Use of the tricyclic compound described in any of Claims 45 to 48 for the manufacture of an antidiarrheal.

77. Use of the tricyclic compound described in any of Claims 45 to 48 for the manufacture of a laxative.
